# EUROPEAN PATENT APPLICATION

(11) **EP 4 155 316 A1**
(43) Date of publication of application: **29.03.2023**
(21) Application number: 21808872.2
(22) Date of filing: 17.05.2021
(51) Int. Cl.: C07K 14/54, C07K 14/715, C07K 16/28, C12N 15/13

(54) **HUMAN IL-15 MUTANT AND USE THEREOF**

(30) Priority: 18.05.2020 CN 202010417427; 30.04.2021 CN 202110483653
(71) Applicant: Simcere (Shanghai) Pharmaceutical Co., Ltd., Shanghai 201321 (CN)
(72) Inventor: DENG, Jing, Nanjing, Jiangsu 210042 (CN); LU, Shiqiang, Nanjing, Jiangsu 210042 (CN); LIU, Yang, Nanjing, Jiangsu 210042 (CN); CAO, Zhuoxiao, Nanjing, Jiangsu 210042 (CN); TANG, Renhong, Nanjing, Jiangsu 210042 (CN); REN, Jinsheng, Nanjing, Jiangsu 210042 (CN)
(74) Representative: KATZAROV S.A.
(86) International application number: PCT/CN2021/094167
(87) International publication number: WO 2021/233260

(57) **Abstract**

Provided are a human IL-15 molecule mutant and a fusion protein containing the IL-15 mutant and combined mutations, wherein the fusion protein can mediate the activation and amplification of immune cells and can be used for treating tumor disease

## Description

### TECHNICAL FIELD

The present disclosure relates to a human IL-15 mutant, a nucleic acid encoding the same, a fusion protein and a pharmaceutical composition comprising the IL-15 mutants and combinated mutations, and related use of the pharmaceutical composition for treating tumors.

### BACKGROUND

Interleukin-15 (IL-15) is an important soluble cytokine discovered and named by Grabstein in detecting the culture supernatant of monkey kidney intradermal cell line CV-1/EBNA in 1994. IL-15 is expressed in a variety of cells and tissues, such as monocytes/macrophages, lymphocytes, epithelial cells, etc.

### Biological Activity of IL-15

### IL-15 mediated signaling pathway

Interleukin 15 receptor (IL15R): IL15R belongs to the hematopoietic factor superfamily and is composed of three subunits: α, β (also known as CD122), and γ (also known as CD132, common gamma chain, γc). IL2 and IL15 share β-chain receptors. IL2, IL4, IL7, IL9, IL15 and IL21 share γ-chain receptors. The receptors for IL2 and IL15 share β- and yc-chains, but both IL2 and IL15 have their respective specific α receptor chains. Human IL15Rα is a type I transmembrane protein. **Both IL2Rα and IL15Rα have a conserved protein-binding motif (sushi domain).** IL15 has a high affinity for II,lSRa (Kd ~ 10⁻¹¹ M), but their binding does not transmit signals. IL15 has moderate affinity for the IL15βγ heterodimer (Kd ~ 10⁻⁹ M), and their binding can transmit signals; the affinity of IL15 and IL15αβγ heterotrimer is similar to that of IL15βγ heterodimer (Kd ~ 10⁻⁹ M), and their binding can transmit signals. Since the receptors of IL2 and IL15 share β and γ chains, IL2 has many similar biological functions as IL15, such as promoting the proliferation of T cells and NK cells.

Binding of IL15 to receptors: IL15Rα is mainly expressed in DCs and monocytes. In most cases, IL15/IL15Rα binds to the receptor in a trans-presented form. That is, after IL-15 and IL-15Ra are expressed in the same cells, IL-15 binds to the sushi domain of IL-15Rα with high affinity in the cells before transporting to the membrane surface and then binds to the βγ heterodimer complex or αβγ heterotrimer complex on the membrane surface of its reactive cells (e.g., T cells or NK cells). β and γ receptors can activate downstream Jak1 and Jak3, respectively, resulting in STAT-3 and STAT-5 activation, activating the cascade, and inducing specific gene expression. When IL15 acts on effector cells in an autocrine form, it can interact with the IL15 receptors in the form of cis and activate downstream signals to produce effector functions.

### Immunomodulatory effects of IL-15

IL-15 has broad immunomodulatory effects and is involved in regulating activity, proliferation and function of a variety of immune cells. (1) Regulatory effects on T cells: IL-15 promotes the activation and proliferation of T cells, promotes the production of memory CD8⁺ T cells, and also plays an important role in maintaining the number of memory CD8⁺ T cells *in vivo.* Even in the presence of Treg cells, IL-15 can well maintain the function and number of CD8⁺ T cells. (2) Regulatory effects on NK cells: IL-15 plays an important role in the activation and proliferation of NK cells and can improve the ADCC killing ability of NK cells. (3) Regulatory effects on other immune cells: IL-15 also plays an important role in the functional maturation of DCs and macrophages. IL-15 can promote the expression of costimulators and IFN-γ by DCs and improve the ability of DCs to activate CD8⁺ T cells and NK cells. In addition, IL-15 can promote neutrophil proliferation.

### Antitumor Effects of IL-15

IL-15 achieves anti-tumor effects based on its ability to expand and activate multiple immune cells, and IL-15 has demonstrated excellent anti-tumor effect in clinical studies. However, due to the short half-life, small molecule size and high renal clearance of wild type IL-15, it is extremely inconvenient to inject it several times a day or subcutaneously. Therefore, the use of wild-type recombinant IL-15 alone is also limited in tumor therapy.
It has been shown that reducing the activity of IL-15 on T cell and NK cell proliferation can increase the half-life of IL-15 while reducing its toxicity. In addition, fusion proteins composed of IL-15 and antibodies targeting tumor-associated antigens can increase the specificity of IL-15, increase the concentration of IL-15 in the tumor microenvironment, and reduce IL-15 toxicity. Therefore, developing IL-15 mutants with reduced activity has the potential to improve the dose-response relationship of IL-15 in the treatment of tumors and expand the clinical anti-tumor application of IL-15, which is of great social and economic significance.

### SUMMARY

The present disclosure provides an IL-15 mutant, a nucleic acid encoding the mutant, a fusion protein and a pharmaceutical composition comprising the mutant, and their functions for killing tumor cells and use for treating tumors.

In a first aspect, the present disclosure provides an IL-15 mutant polypeptide comprising mutation(s) at one or more amino acid residues corresponding to Val3, Ile6, Asp8, or His105 of wild-type IL-15.

In a second aspect, the present disclosure provides a polypeptide comprising an IL-15 mutant, wherein the polypeptide comprises mutation(s) at one or more amino acid residues corresponding to Val3, Ile6, Asp8, or His105 of wild-type IL-15.

In one embodiment, the IL-15 mutant polypeptide comprises at least mutations at two, three, or four amino acid residues at Val3, Ile6, Asp8, or His105.

In one embodiment, the mutation is a substitution, insertion or deletion.

In a specific embodiment, the mutation is selected from amino acid substitution of the group of: Val3Leu (V3L), Ile6Asp (I6D), Ile6Pro (I6P), Asp8Glu (D8E), Asp8Gln (D8Q), Asp8Arg (D8R), Asp8Ser (D8S), Asp8Val (D8V), Asp8Gly (D8G), Asp8Ile (D8I), Asp8Leu (D8L), Asp8Thr (D8T), His105Asn (H105N), and/or His105Lys (H105K).

In a specific embodiment, the IL-15 mutant polypeptide or the polypeptide comprising an IL-15 mutant comprises mutation or combination of mutations of: (1) Asp8Glu, (2) Asp8Gln, (3) Asp8Arg, (4) Asp8Ser, (5) Asp8Val, (6) Val3Leu, (7) Ile6Asp, (8) His105 Lys, (9) His105 Asn, (10) Asp8Gly, (11) Asp8Ile, (12) Asp8Leu, (13) Ile6Pro, (14) Asp8Thr, (15) Asp8Glu and Val3Leu, (16) Asp8Glu and Ile6Asp, (17) Val3Leu and Ile6Asp, (18) Ile6Asp and His105Lys, (19) Asp8Ser and His105Lys, (20) Asp8Ser and His105Asn, or (21) Val3Leu, Ile6Asp and His105Lys.

In a specific embodiment, the IL-15 mutant has at least 80%, 85%, 90%, 91%, 92%, 93%, 94%, 95%, 96%, 97%, 98%, or 99% sequence identity to human wild-type IL-15.

In a specific embodiment, the amino acid sequence of the IL-15 mutant is as shown in SEQ ID NO.3, SEQ ID NO.5, SEQ ID NO.7, SEQ ID NO.9, SEQ ID NO.11, SEQ ID NO. 13, SEQ ID NO.15, SEQ ID NO.17, SEQ ID NO.19, SEQ ID NO.21, SEQ ID NO.23, SEQ ID NO.25, SEQ ID NO.27, SEQ ID NO.29, SEQ ID NO.35, SEQ ID NO.37, SEQ ID NO.39, SEQ ID N0.41, SEQ ID NO.43, SEQ ID NO.45 or SEQ ID NO.47.

In a specific embodiment, the IL-15 mutant polypeptide or the polypeptide comprising an IL-15 mutant has activities of: (1) mediating proliferation of human CD8⁺ T cells; (2) mediating proliferation of human NK cells; and/or (3) inhibiting tumor growth.

In a specific embodiment, the IL-15 mutant polypeptide or the polypeptide comprising an IL-15 mutant has activity of mediating proliferation/expansion of CD8⁺ T cells and/or NK cells which is lower than that of a polypeptide comprising a wild-type IL-15.

In a specific embodiment, the wild-type IL-15 has an amino acid sequence as shown in SEQ ID NO. 1.

In a third aspect, the present disclosure provides a protein comprising the aforementioned IL-15 mutant polypeptide or the polypeptide comprising an IL-15 mutant; wherein the protein further comprises an immunoglobulin molecule or part thereof fused to the IL-15 mutant, and/or IL-15Rα fused to the IL-15 mutant.

In one embodiment, the immunoglobulin molecule is an antibody or antigen-binding fragment; part of the immunoglobulin molecule is an immunoglobulin Fc region.

In one embodiment, the antibody or antigen-binding fragment is selected from: (1) a chimeric antibody or fragment thereof; (2) a humanized antibody or fragment thereof; or (3) a fully humanized antibody or fragment thereof.

In a specific embodiment, the antibody or antigen-binding fragment is selected from one or more of F(ab)₂, Fab', Fab, Fv, scFv, bispecific antibody, nanobody and antibody minimum recognition unit.

In a specific embodiment, the immunoglobulin Fc region is selected from a Fc region of any one of IgG1, IgG2, IgG3, IgG4, IgA, IgM, IgE or IgD; preferably, a sequence comprising a constant region of human or murine IgG1, IgG2, IgG3 or IgG4 antibody; and preferably, the immunoglobulin Fc region has an amino acid sequence as shown in SEQ ID NO.73.

In another embodiment, the IL-15 mutant is fused to the immunoglobulin molecule or part thereof with or without a linker peptide, or the IL-15 mutant is fused to the IL-15 Rα with or without a linker peptide; preferably, a linker peptide is used; and preferably, the linker peptide as shown in SEQ ID NO.65, SEQ ID NO.67, SEQ ID NO.69, or SEQ ID NO.71 is used.

In another embodiment, the IL-15 mutant is fused to the IL-15Rα and then to the immunoglobulin molecule or part thereof with or without a linker peptide; preferably, a linker peptide is used; and preferably, the linker peptide as shown in SEQ ID NO.65, SEQ ID NO.69, or SEQ ID NO.71 is used.

In a specific embodiment, individual domains are connected from N-terminus to C-terminus is in order of:
(1) the immunoglobulin protein molecule or part thereof, the IL-15Rα, and the IL-15 mutant;
(2) the immunoglobulin molecule or part thereof, the IL-15 mutant, and the IL-15Rα;
(3) the IL-15 mutant, the IL-15Rα, and the immunoglobulin molecule or part thereof;
(4) the IL-15Rα, the IL-15 mutant, and the immunoglobulin molecule or part thereof;
(5) the IL-15 mutant, and the immunoglobulin molecule or part thereof;
(6) the immunoglobulin molecule or part thereof, and the IL-15 Rα;
(7) the IL-15Rα, and the immunoglobulin molecule or part thereof;
(8) the IL-15 mutant, and the IL-15Rα; or,
(9) the IL-15Rα, and the IL-15 mutant.

In another embodiment, the IL-15Rα or IL-15 mutant is fused to N-terminus of a variable region of a heavy chain of the immunoglobulin molecule or C-terminus of the immunoglobulin Fc region when the IL-15Rα or IL-15 mutant is fused to the immunoglobulin molecule; and the IL-15Rα or IL-15 mutant is fused to N-terminus or C-terminus of the immunoglobulin Fc region when the IL-15Rα or IL-15 mutant is fused to the immunoglobulin Fc region.

In a fourth aspect, the present disclosure provides a protein or an antibody fusion construct/complex comprising:
(1) an immunoglobulin heavy chain;
(2) an immunoglobulin light chain;
(3) an IL-15Rα; and,
(4) the IL-15 mutant polypeptide as described in the first or second aspect.

In one embodiment, the IL-15Rα is fused to N-terminus of a variable region of the immunoglobulin heavy chain or C-terminus of an immunoglobulin Fc region with or without a linker peptide.

In one embodiment, the IL-15 mutant polypeptide is non-covalently linked to the IL-15Rα, or the IL-15 mutant is fused to the other end of the IL-15Rα with or without a linker peptide.

In a specific embodiment, the protein is a homodimer comprising a monomer composed of (1) to (4).

In a fifth aspect, the present disclosure provides a protein or Fc fusion construct comprising:
(1) an immunoglobulin Fc region;
(2) an IL-15Rα; and,
(3) the IL-15 mutant polypeptide as described in the first or second aspect.

In one embodiment, the IL-15Rα is fused to N- or C-terminus of the IL-15 mutant polypeptide with or without a linker peptide, and then fused to N- or C-terminus of the immunoglobulin Fc region with or without a linker peptide.

In a specific embodiment, the protein is a homodimer comprising a monomer composed of (1) to (3).

In another preferred embodiment, the immunoglobulin is selected from an anti-PD-L1 antibody; preferably, the anti-PD-L1 antibody is Tecentriq, KN-035, or 794-h1-71.

In a specific embodiment, the anti-PD-L1 antibody comprises a heavy chain variable region and a light chain variable region, wherein the heavy chain variable region and the light chain variable region have sequences shown in SEQ ID NO: 99 and SEQ ID NO: 100, respectively, or sequences having at least 80%, 85%, 90%, 95%, 96%, 97%, 98%, 99%, or greater identity to the sequences shown in SEQ ID NO: 99 and SEQ ID NO: 100; or the heavy chain variable region and light chain variable region have sequences shown in SEQ ID NO: 97 and SEQ ID NO: 98, respectively, or sequences having at least 80%, 85%, 90%, 95%, 96%, 97%, 98%, 99% or greater identity to the sequences shown in SEQ ID NO: 97 and SEQ ID NO: 98.

In another preferred embodiment, the IL-15Rα is selected from an IL-15Rα-sushi; preferably, the IL-15Rα-sushi has an amino acid sequence as shown in SEQ ID NO.49, SEQ ID NO.51, SEQ ID NO 53, or SEQ ID NO.55.

In a sixth aspect, the present disclosure provides an antibody or antigen-binding fragment capable of specifically binding to PD-L1, wherein the antibody or antigen-binding fragment comprises a heavy chain variable region and a light chain variable region; preferably, the heavy chain variable region and the light chain variable region have sequences shown in SEQ ID NO: 99 and SEQ ID NO: 100, respectively, or sequences having at least 80%, 85%, 90%, 95%, 96%, 97%, 98%, 99% or greater identity to the sequences shown in SEQ ID NO: 99 and SEQ ID NO: 100.

In a specific embodiment, the antibody or antigen binding fragment binds to human programmed death-ligand 1 (PD-L1) at a dissociation constant (KD) of 1.8 × 10⁻⁹ M or less, and the antibody or antigen binding fragment binds to cynomolgus monkey programmed death - ligand 1 (PD-L1) at a dissociation constant (KD) of 9.4 × 10⁻¹⁰ M or less; or, optionally, the antibody or antigen-binding fragment binds to or does not bind to monkey PD-L1; optionally, the antibody or antigen-binding fragment binds to or does not bind to murine PD-L1.

In a specific embodiment, the anti-PD-L1 antibody competitively binds to PD-L1 or epitope thereof, and has activities of:
(1) specifically binding to a recombinant PD-L1 protein and a cell expressing PD-L1;
(2) blocking binding of PD-L1 to PD-1 protein;
(3) inhibiting binding of PD-1 to PD-L1 expressed on cell surface;
(4) enhancing T cell activity; or/and
(5) inhibiting tumor growth.

In a preferred embodiment, the anti-PD-L1 antibody comprises a constant region derived from any one of IgG1, IgG2, IgG3, IgG4, IgA, IgM, IgE, or IgD; preferably, a sequence comprising a constant region of human or murine IgG1, IgG2, IgG3, or IgG4 antibody.

In another preferred embodiment, the anti-PD-L1 antibody is selected from one or more of F(ab)₂, Fab', Fab, Fv, scFv, and bispecific antibody.

In a seventh aspect, the present disclosure provides an isolated nucleic acid molecule encoding the polypeptide, protein, antigen or antigen-binding fragment described in any one of the preceding first to sixth aspects.

In an eighth aspect, the present disclosure provides an expression vector comprising the isolated nucleic acid molecule of the seventh aspect.

In a ninth aspect, the present disclosure provides a host cell comprising the isolated nucleic acid molecule of the seventh aspect, or the expression vector of the eighth aspect; preferably, the host cell is a eukaryotic or prokaryotic cell; more preferably, the host cell is derived from mammal cells, yeasts, insects, *Escherichia coli* and/or *Bacillus subtilis;* more preferably, the host cell is selected from Chinese hamster ovary (CHO) cells.

In a tenth aspect, the present disclosure provides a method for preparing a polypeptide or protein, comprising culturing the host cell of the ninth aspect under an appropriate condition, and isolating the polypeptide or protein.

In an eleventh aspect, the present disclosure provides a pharmaceutical composition comprising the polypeptide, protein, antigen or antigen-binding fragment described in any one of the preceding first to sixth aspects, the isolated nucleic acid molecule of the preceding seventh aspect, the expression vector of the preceding eighth aspect, the host cell of the ninth aspect, or the product prepared by the method of the tenth aspect; and a pharmaceutically acceptable carrier; preferably, the pharmaceutical composition further comprises an additional anti-tumor agent.

In a twelfth aspect, the present disclosure provides use of the polypeptide, protein, antigen or antigen-binding fragment described in any one of the preceding first to sixth aspects, the isolated nucleic acid molecule of the preceding seventh aspect, the expression vector of the preceding eighth aspect, the host cell of the ninth aspect, the product prepared by the method of the tenth aspect, or the pharmaceutical composition of the eleventh aspect in the manufacture of a medicament for the prevention and/or treatment of a disease in an individual; preferably, the disease is a tumor.

In a thirteenth aspect, the present disclosure provides a method for preventing and/or treating a disease in an individual, comprising administering to a patient in need thereof the polypeptide, protein, antigen or antigen-binding fragment described in any one of the preceding first to sixth aspects, the isolated nucleic acid molecule of the preceding seventh aspect, the expression vector of the preceding eighth aspect, the host cell of the ninth aspect, the product prepared by the method of the tenth aspect, or the pharmaceutical composition of the eleventh aspect; preferably, the disease is a tumor.

### Terms and Definitions

Unless otherwise specified, terms used herein have meanings commonly understood by those skilled in the art. For terms expressly defined herein, the meanings of such terms shall be subject as defined herein.

As used herein, the term "IL-15", "IL15" or "interleukin-15 (IL-15)" refers to a pleiotropic cytokine that activates T cells, B cells and NK cells and mediates the proliferation and survival of these cells. In addition, IL-15 can activate, maintain and expand CD8⁺ memory T cells. The "IL-15" or "IL-15 peptide fragment" or "IL-15 polypeptide" according to the present disclosure can be any IL-15 (interleukin 15) or a mutant thereof, such as human IL-15 or non-human mammal IL-15 or non-mammalian IL-15. Exemplary non-human mammals are such as pigs, rabbits, monkeys, orangutans, mice, etc., non-mammals are such as chickens, and the like. Preferably, human interleukin 15 mature molecule, see the database UniProtKB, Login ID P40933, 49-162aa.

As used herein, the term "IL-15 wild-type" or "wild-type IL-15" refers to human IL-15 from natural sources or non-human mammalian IL-15 or non-mammalian IL-15; It can also refer to the IL-15 polypeptides that have been commonly used in the art.

As used herein, the term "IL-15 mutant" refers to a mutant molecule with increased or decreased affinity to IL-15 receptors or with increased or decreased activity in T cell or NK cell proliferation, and cytokine release when stimulating a specific cell line, wherein the mutant molecule is obtained by one or more amino acid substitutions, additions or deletions.

As used herein, the term "IL-15Rα" may refer to IL-15Rα of any species or a functional fragment thereof, such as human IL-15Rα or non-human mammalian IL-15Rα or non-mammalian IL-15Rα. Exemplary non-human mammals are such as pigs, rabbits, monkeys, orangutans, mice, etc., and non-mammals such as chickens, and the like. Preferably, human IL-15Rα; preferably, human interleukin 15 receptor α extracellular domain fragment, referred to as IL-15Rα ECD; preferably, IL-15Rα-sushi, see Table 1 for details.

As used herein, the term "IL-15Rα variant" refers to a functional mutant formed by one or more amino acid deletion, insertion or substitution mutations on IL-15Rα, which has the ability to bind to its ligand molecule such as IL15, preferably human IL15Rα molecules, more preferably a shortened form of human IL-15Rα extracellular domain fragment, that is, a molecule with human interleukin 15 receptor α activity obtained by one or more amino acid deletion mutations from the C-terminus of the extracellular domain fragment, preferably a deletion mutation form with 65-120 amino acids retained, more preferably a shortened form of deletion mutation with 65-102 amino acids retained, such as IL-15Rα-sushi; preferably IL-15Rα-sushi, see Table 3 for details.

As used herein, the term "immunoglobulin Fc region" refers to the constant region of an immunoglobulin chain, especially the carboxyl terminus of the constant region of an immunoglobulin heavy chain or a part thereof, which has no antigen-binding activity and is the site where antibody molecules interact with effector molecules and cells. The "immunoglobulin Fc region" of the present disclosure can be any Fc or a variant thereof, which is derived from human or non-human mammals. For example, an immunoglobulin Fc region can include two or more domains of heavy chains CH1, CH2, CH3, CH4 in combination with an immunoglobulin hinge region. Fc can be derived from different species, preferably human immunoglobulins. According to the amino acid sequence of the heavy chain constant region, immunoglobulins can be divided into different classes, mainly including five types of immunoglobulins: IgA, IgD, IgE, IgG and IgM. Some of these can be further divided into subclasses (isotypes), such as IgG-1, IgG-2, IgG-3, IgG-4; IgA-1 and IgA-2. The "Fc region" preferably comprises at least one immunoglobulin hinge region, as well as the CH2 and CH3 domains of IgG. More preferably, it comprises a CH2 domain, a CH3 domain and an immunoglobulin hinge region of IgG1, and the starting amino acid position of the hinge region can be varied.

As used herein, the term "Fc variant" refers to a change in Fc structure or function caused by one or more amino acid substitution, insertion or deletion mutations at a suitable site on Fc. "Interaction between Fc variants" refers to space-filling effects, electrostatic guidance, hydrogen bonding, hydrophobic interactions, and the like formed between Fc variants designed by mutation. Interaction between Fc variants contributes to the formation of stable heterodimeric proteins. Preferred mutagenesis designs are those of the "Knob-into-Hole" form.

The mutation design technology of Fc variants has been widely used in the art to prepare bispecific antibodies or heterodimeric Fc fusion proteins. The representative is the "Knob-into-Hole" form proposed by Cater et al. (Protein Engineering vol. 9 no. 7 pp. 617-621, 1996); Fc-containing heterodimeric form formed by Amgen's technicians using electrostatic guidance (Electrostatic Steering) (US 20100286374 A1); the heterodimeric form (SEED bodies) formed by IgG/IgA chain exchange proposed by Jonathan H. Davis et al. (Protein Engineering, Design & Selection pp. 1-8, 2010); a bispecific molecule formed by Genmab's DuoBody (Science, 2007.317(5844)) platform technology; a heterodimeric protein form formed by Xencor's technical staff integrating structural calculations and Fc amino acid mutations, and integrating different modes of action (mAbs 3:6, 546-557; November/December 2011); the form of heterodimeric protein obtained by the method of Fc transformation based on charge network (CN201110459100.7) of Alphamab Company ; and other methods based on Fc amino acid changes or functional transformation to achieve the genetic engineering of the heterodimeric functional protein. The Knob/Hole structure in the Fc variant fragment of the present disclosure refers to the mutation of the two Fc fragments respectively, which can be combined in the form of "Knob-into-Hole" after the mutation. It is preferred to use the "knob-into-hole" model of Cater et al. to carry out site mutation engineering in the Fc region, so that the first Fc variant and the second Fc variant can be combined in the form of "knob into hole" to form a heterodimer. The selection of a particular immunoglobulin Fc region from a particular immunoglobulin class and subclass is within the purview of those skilled in the art. The Fc region of human antibody IgG1, IgG2, IgG3, and IgG4 is preferred, and the Fc region of human antibody IgG1 is more preferred. One of the first Fc variant or the second Fc variant is randomly selected to undergo the knob mutation and the other to undergo the hole mutation.

As used herein, the term "antibody (Ab)" refers to an immunoglobulin molecule that specifically binds to the target antigen or has immunoreactivity, including polyclonal, monoclonal, genetically engineered and other modified forms of antibodies (including but not limited to chimeric antibodies, humanized antibodies, fully humanized antibodies, heterologous coupled antibodies (such as bispecific, trispecific and tetraspecific antibodies, diabodies, tribodies and tetrabodies, and antibody conjugates) and antigen-binding fragments of antibodies (including, for example, Fab', F(ab')2, Fab, Fv, rIgG and scFv fragments). In addition, unless otherwise defined, the term "monoclonal antibody" (mAb) is intended to include intact antibody molecules as well as incomplete antibody fragments (e.g. Fab and F(ab')2 fragments, which lack the Fc fragment of the intact antibody (cleansed more quickly from the animal circulation) and therefore lack Fc-mediated effector function) capable of specifically binding to a target protein (see Wahl et al., J. Nucl. Med. 24: 316, 1983; the content of which are incorporated herein by reference).

As used herein, the term "humanized antibody" refers to a genetically engineered non-human antibody whose amino acid sequence has been modified to increase homology to the sequence of a human antibody. Typically, all or part of the CDR regions of a humanized antibody are derived from non-human antibodies (donor antibodies), and all or part of the non-CDR regions (e.g., variable FR and/or constant regions) are derived from human Immunoglobulins (receptor antibodies). Humanized antibodies generally retain or partially retain the expected properties of the donor antibody, including, but not limited to, antigen specificity, affinity, reactivity, ability to increase immune cell activity, ability to enhance immune response, and the like.

The term "antibody conjugate" refers to a couplet/conjugate formed by the chemical bonding of an antibody molecule either directly or via a linker to another molecule. For example, an antibody-drug conjugate (ADC), in which the drug molecule is another molecule described.

The term "monoclonal antibody" refers to an antibody derived from a single clone (including any eukaryotic, prokaryotic, or phage clones) without limitation to the method of production of the antibody.

As used herein, the term "fusion protein" refers to a protein product obtained by connecting the coding regions of two or more genes through gene recombination method, chemical method or other appropriate methods and expressing recombined gene under the control of the same regulatory sequence. In the fusion protein of the present disclosure, the coding regions of two or more genes can be fused at one or several positions by sequences encoding peptide linkers or linker peptides. Peptide linkers or linker peptides can also be used to construct fusion proteins of the present disclosure. The term "fusion protein" of the present disclosure further includes antibody/Fc fusion protein constructs/complexes, or compositions of antibody/Fc fusion protein constructs/complexes formed by non-covalent means, for example, the fusion protein described in the present disclosure can be shown as the following structure:
(1) IL-15 fusion protein, which is a homodimer comprising two monomers; the monomers comprise an antibody heavy chain, an antibody light chain, an IL-15 and an IL-15Rα sushi; for example, the antibody heavy chain Fc is fused to IL-15Rα sushi, and co-expressed with the antibody light chain, and IL-15-WT (wild type) or IL-15 mutant, so that IL-15 and IL-15Rα sushi form a non-covalent link;
(2) IL-15 fusion protein, which is a homodimer comprising two monomers; the monomers comprise an antibody heavy chain, an antibody light chain, an IL-15 and an IL-15Rα sushi; for example, the antibody heavy chain Fc is fused and expressed in series with IL-15Rα sushi and IL-15-WT (wild type) or IL-15 mutant by a linker, and co-expressed with the antibody light chain;
(3) IL-15 fusion protein, which is a homodimer comprising two monomers; the monomers comprise a Fc, an IL-15, and an IL-15Rα sushi; for example, IL-15-WT or IL-15 mutant is linked to IL15-Rα sushi by a linker, and then IL15-Rα sushi is linked to Fc by a linker; or,
(4) IL-15 fusion protein, which is a homodimer comprising two monomers; the monomers comprise a Fc, an IL-15 and an IL-15Rα sushi; for example, IL15-Rα sushi is linked to IL-15 - WT or IL-15 mutants by a linker, and then IL-15 is linked to Fc by a linker.

As used herein, the term "peptide linker/linker peptide/linker" refers to a peptide used in the present disclosure to link IL-15 with another protein molecule or protein fragment to ensure the correct folding and stability of the protein. Said another molecule includes, but is not limited to, IL-15Rα, Fc, Fc variant, antibody, and the like. The "linker" of the present disclosure is preferably (GGGGS)ₙ, wherein n can be 0, 1, 2, 3, 4, 5 or more, preferably n is 2-4; or preferably SGGSGGGGSGGGSGGGGSLQ. If the linker sequence is too short, it may affect the folding of the higher-order structures of the two proteins, thereby interfering with each other; if the linker sequence is too long, it will involve the problem of immunogenicity, because the linker sequence itself is a new antigen.

As used herein, the term "heterodimeric protein" refers to a protein formed by combining two different monomeric proteins. In the present disclosure, two different monomeric proteins each contain an Fc fragment or an Fc variant fragment, and form a heterodimeric protein by interaction of the Fc fragment or the Fc variant fragment.

As used herein, the term "homodimeric protein" refers to a protein formed by combining two identical monomeric proteins. In the present disclosure, two identical monomeric proteins each contain an Fc fragment or an Fc variant fragment, and form a homodimeric protein through the interaction of the Fc fragment or the Fc variant fragment.

The "monomeric protein" constituting the heterodimeric protein or the homodimeric protein in the present disclosure may be a fusion protein or a non-fusion protein.

As used herein, the term "PD-L1" refers to programmed death ligand-1, also known as CD279 (differentiation cluster 279), which is an important immunosuppressive molecule. The PD-L1 is preferably human PD-L1.

As used herein, the term "anti-programmed death ligand-1 antibody", "programmed death ligand-1 antibody", "anti-PD-L1 antibody", "PD-L1 antibody", "anti-PD-L1 antibody moiety" and/or "anti-PD-L1 antibody fragment" and the like refer to any protein- or peptide-containing molecules comprising at least a part of an immunoglobulin molecule capable of specifically binding to PD-L1 (for example, but not limited to at least one complementarity determining region (CDR) of a heavy or light chain or a ligand binding part thereof, a heavy or light chain variable region, a heavy or light chain constant region, a framework region or any part thereof). The PD-L1 antibody also includes an antibody-like protein scaffold (such as the tenth fibronectin type III domain (10Fn3)), which contains BC, DE and FG structural loops similar in structure and solvent accessibility to CDR of the antibody. The tertiary structure of the 10Fn3 domain is similar to the tertiary structure of the heavy chain variable region of IgG, and by replacing the residues of the BC, DE and FG loops of 10Fn3 with residues of the CDR-H1, CDR-H2 or CDR-H3 region from the PD-L1 monoclonal antibody, one skilled in the art can graft, for example, the CDR of the PD-L1 monoclonal antibody onto the fibronectin scaffold.

As used herein, the term "co-expression" refers to the expression of multiple genes together in a cell and the simultaneous appearance of their products. These genes can co-exist and be expressed individually or jointly under control. In the present disclosure, two genes are preferably co-expressed in one eukaryotic cell. The gene expression product obtained by co-expression is favorable for the efficient and simple formation of complexes; in the present disclosure, it is favorable for the formation of a heterodimeric protein or a homodimeric protein.

As used herein, the term "percent (%) sequence identity" refers to the percentage of amino acid (or nucleic acid) residues of a candidate sequence that are identical to the amino acid (or nucleic acid) residues of a reference sequence after aligning the sequences and introducing gaps, if necessary, to achieve the maximum percent sequence identity (e.g., gaps can be introduced in one or both of the candidate and reference sequences for optimal alignment and non-homologous sequences can be disregarded for comparison purposes). Alignment for purposes of determining percent sequence identity can be achieved in various ways that are well known to those skilled in the art, for instance, using publicly available computer software, such as BLAST, ALIGN, or Megalign (DNASTAR) software. Those skilled in the art can determine appropriate parameters for measuring alignment, including any algorithms needed to achieve maximal alignment over the full length of the sequences being compared. For example, a reference sequence aligned for comparison with a candidate sequence may show that the candidate sequence exhibits from 50% to 100% sequence identity across the full length of the candidate sequence or a selected part of contiguous amino acid (or nucleic acid) residues of the candidate sequence. The length of the candidate sequence aligned for comparison purposes may be, e.g., at least 30%, (e.g., 30%, 40, 50%, 60%, 70%, 80%, 90%, or 100%) of the length of the reference sequence. When a position in the candidate sequence is occupied by the same amino acid residue as the corresponding position in the reference sequence, then the molecules are identical at that position.

As used herein, that term "specific binding" refers to a binding reaction that determines the presence of an antigen in a heterogeneous population of proteins and other biomolecules which are specifically recognized, for example, by antibodies or antigen-binding fragments thereof. An antibody or antigen-binding fragment thereof that specifically binds to an antigen will bind to the antigen with a KD of less than 100nM. For example, an antibody or antigen-binding fragment thereof that specifically binds to an antigen will bind to the antigen with a KD of up to 100nM (e.g., between 1pM and 100nM). An antibody or antigen-binding fragment thereof that does not show specific binding to a particular antigen or epitope thereof will show a KD of greater than 100nM (e.g., greater than 500nM, 1µM, 100µM, 500µM or 1mM) for that particular antigen or epitope thereof. A variety of immunoassay methods can be used to select antibodies that show a specific immune response against specific proteins or carbohydrates. For example, solid-phase ELISA immunoassay is routinely used to select antibodies that shows a specific immune response against proteins or carbohydrates. See Harlow & Lane, Antibodies, ALabortory Manual, Cold Spring Harbor Press, NewYork (1988) and Harlow & Lane, Using Antibodies, A Laboratory Manual, Cold Spring Harbor Press, NewYork (1999), which describe immunoassay methods and conditions that can be used to determine specific immunoreactivity.

As used herein, the term "vector" includes a nucleic acid vector, such as a DNA vector (e.g., a plasmid), an RNA vector, a virus, or other suitable replicon (e.g., a viral vector). Various vectors have been developed for delivering polynucleotides encoding foreign proteins into prokaryotic or eukaryotic cells. The expression vector of the present disclosure contains polynucleotide sequences and additional sequence elements, for example, for expressing proteins and/or integrating these polynucleotide sequences into the genome of mammalian cells. Certain vectors that can be used to express antibodies and antibody fragments of the present disclosure include plasmids containing regulatory sequences (such as promoter and enhancer regions) that direct gene transcription. Other useful vectors for expressing antibodies and antibody fragments contain polynucleotide sequences that enhance the translation rate of these genes or improve the stability or nuclear output of mRNA produced by gene transcription. These sequence elements include, for example, 5'and 3' untranslated regions, internal ribosomal entry sites (IRES) and polyadenylation signal sites to direct effective transcription of genes carried by expression vectors. The expression vector of the present disclosure may also contain polynucleotides encoding markers for selecting cells containing such vectors. Examples of suitable markers include genes encoding resistance to antibiotics, such as ampicillin, chloramphenicol, kanamycin or nourseothricin.

As used herein, the terms "subject" and "patient" refer to an organism receiving treatment for a particular disease or condition, such as cancer or infectious disease, as described herein. Examples of subjects and patients include mammals, such as humans, primates, pigs, goats, rabbits, hamsters, cats, dogs, guinea pigs, bovine family members (such as domestic cattle, bison, buffalo, elk, yak, etc.), sheep and horses, receiving treatment for diseases or conditions, such as cell proliferative disorders, for example, cancer or infectious diseases.

As used herein, the term "treatment" refers to surgical or therapeutic treatment, the purpose of which is to prevent, alleviate (reduce) the progression of undesirable physiological changes or lesions in the subject of treatment, such as the progression of cell proliferative disorders (e.g., cancer or infectious diseases). Beneficial or desired clinical outcomes include but not limited to, alleviation of symptoms, reduction of disease severity, stabilization (*i.e*., no deterioration) of the disease state, delay or amelioration of disease progression, improvement or mitigation of the disease state, and remission (whether partial or complete), whether detectable or undetectable. The subjects to be treated include those who already suffer from diseases or conditions, and those who are susceptible to diseases or conditions or intend to prevent diseases or diseases. When referring to the terms such as alleviation, reduction, mitigation, amelioration and remission, the meanings thereof also include elimination, disappearance and non-occurrence.

As used herein, the term "immune disorders" include, for example, pathological inflammations, inflammatory disorders and autoimmune disorders or diseases. "Immune disorders" also refers to infections, persistent infections, and proliferative disorders, such as cancer, tumor, and angiogenesis. "Cancerous disorder" includes, for example, cancer, cancer cell, tumor, angiogenesis, and precancerous condition, such as dysplasia.

As used herein, the term "pharmaceutical composition" refers to a mixture comprising one or more of the compounds described herein, or a physiologically/pharmaceutically acceptable salt or prodrug thereof, with other chemical components, as well as other components such as physiological/pharmaceutically acceptable carriers and excipients. The purpose of the pharmaceutical composition is to facilitate the administration to the organism and facilitate the absorption of the active ingredient to exert its biological activity.

As used herein, that term "Size Exclusion Chromatograph, SEC " refers to a liquid chromatography technique for separation according to the molecular size of the cvn component to be tested. There are pores of different sizes distributed on the surface of the chromatographic column filler. After the sample enters the chromatographic column, different components in the sample enter into the pores of corresponding pore size according to their molecular size. Molecules larger than all pore sizes cannot enter into the filler particles, and will not be retained during the chromatographic process with a short retention time; molecules smaller than all pore sizes can freely enter into the pores of all pore sizes on the surface of the filler with a longer retention time in the chromatographic column, resulting in a longer retention time; the other molecules are eluted in sequence according to the molecular size.

"Optional" or "optionally" means that the subsequently event or environment described can but need not occur, and the description includes the occasions where the event or environment occurs or does not occur. For example, "optionally comprising 1-3 antibody heavy chain variable regions" means that antibody heavy chain variable regions may but need not be present; when the antibody heavy chain variable region(s) presents, there may be 1, 2 or 3 of them.

The steps of transforming host cells with recombinant DNA described in the present disclosure can be performed using conventional techniques well known to those skilled in the art. The obtained transformants can be cultured by conventional methods to express the polypeptides encoded by the genes of the present disclosure. According to the host cells to be used, the medium used in the culture can be selected from various conventional media. The host cells are cultured under conditions suitable for the growth of the host cells.

### BRIEF DESCRIPTION OF THE DRAWINGS

The foregoing and other aspects of the present disclosure will be clearly illustrated by the following detailed description and drawings of the present disclosure. The drawings herein are intended to illustrate some preferred embodiments of the present disclosure, however, it is understood that the present disclosure is not limited to the particular embodiments disclosed.
Figure 1 shows the result of blocking the binding of PD-L1 and PD-1 by humanized PD-L1 antibody, wherein the positive control is Tecentriq.
Figure 2 shows the binding ability of humanized PD-L1 antibody to PD-L1 at the cellular level determined by FACS, wherein the positive control is Avelumab.
Figure 3 shows the ability of humanized PD-L1 antibody to block PD-L1/PD-1 tested by using Jurkat-PD-1/CHO-PD-L1-NFAT system, wherein the positive control is Avelumab.
Figure 4 shows result of IFN-γ secretion in mixed lymphocyte reaction promoted by humanized anti-PD-L1 antibody, wherein the negative control is anti-Hel antibody and the positive control is Avelumab.
Figure 5 shows two structures of IL-15 fusion protein:
   A: IL-15 wild-type or mutant and IL-15Rαsushi are co-expressed and assembled by non-covalent linking;
   B: IL-15 wild-type or mutant and IL-15Rαsushi are fused and assembled in tandem by linker for expression;
   C: IL-15 wild type or mutant is first linked to IL-15Rαsushi by linker, and IL-15Rαsushi and Fc are then fused and assembled in tandem by linker for expression;
   D: IL-15Rαsushi is first linked to IL-15 wild type or mutant by linker, and IL-15 wild type or mutant and Fc are then fused and assembled in tandem by linker for expression.

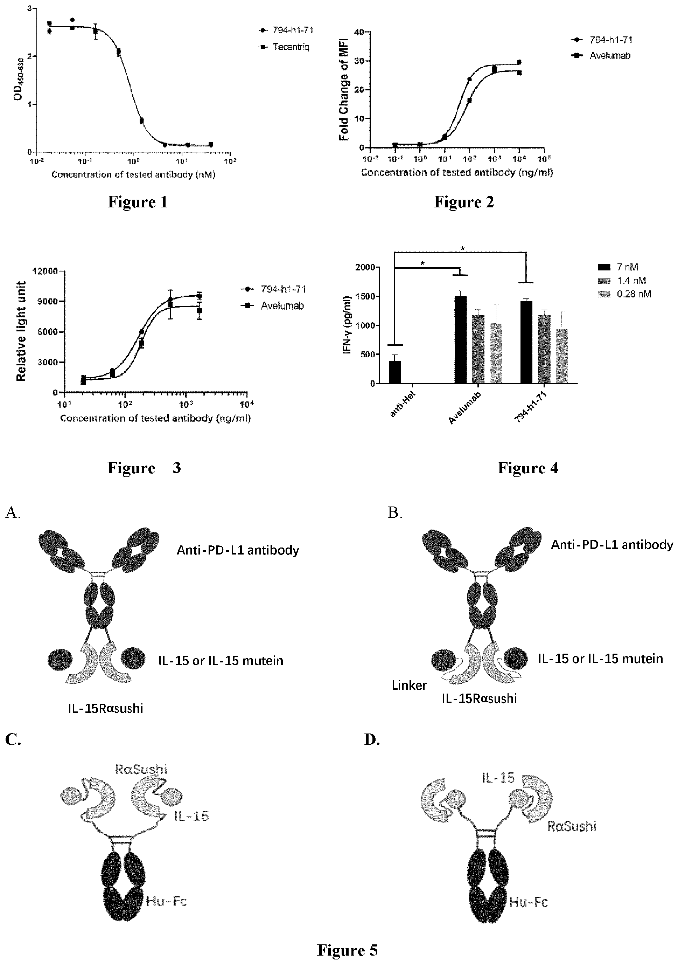

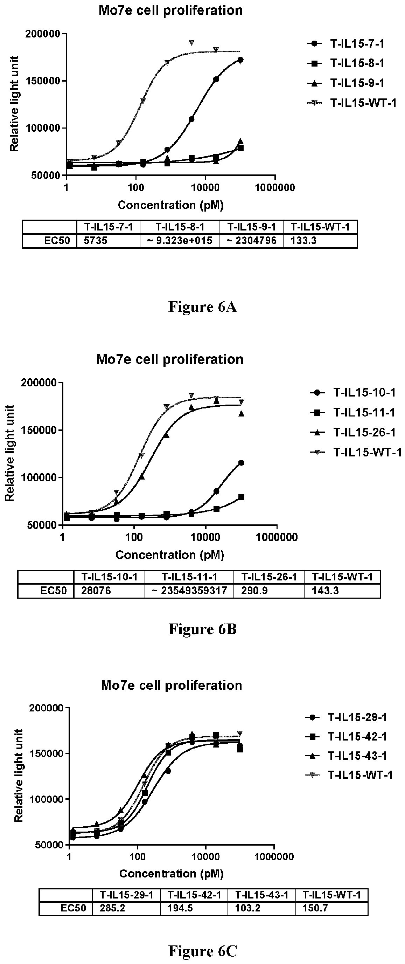

Figures 6A-6D show IL15 mutant promotes Mo7e cell proliferation: the structure of the fusion protein is as shown in Figure 5A; Tecentriq is fused to IL-15Rαsushi, and IL15 mutant is non-covalently linked to IL-15Rαsushi without linker ligation.

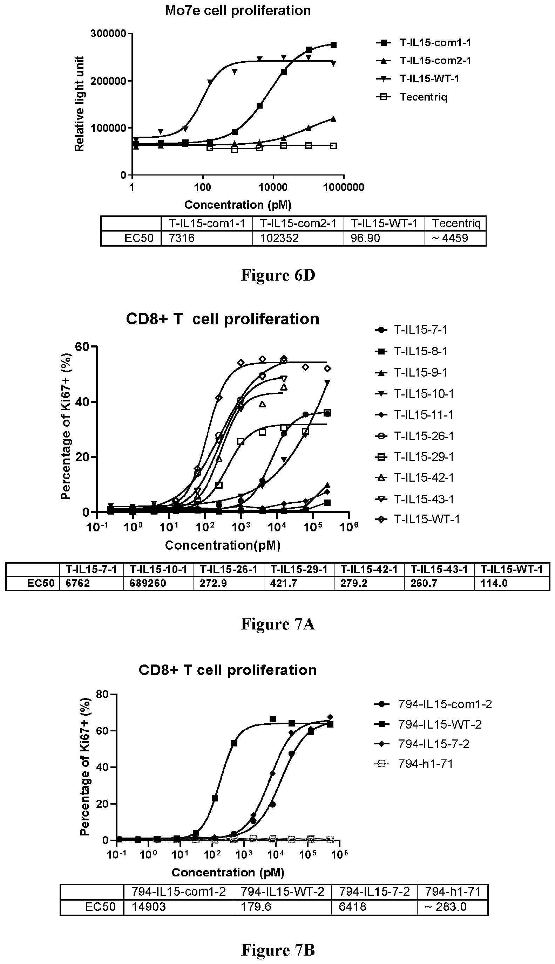

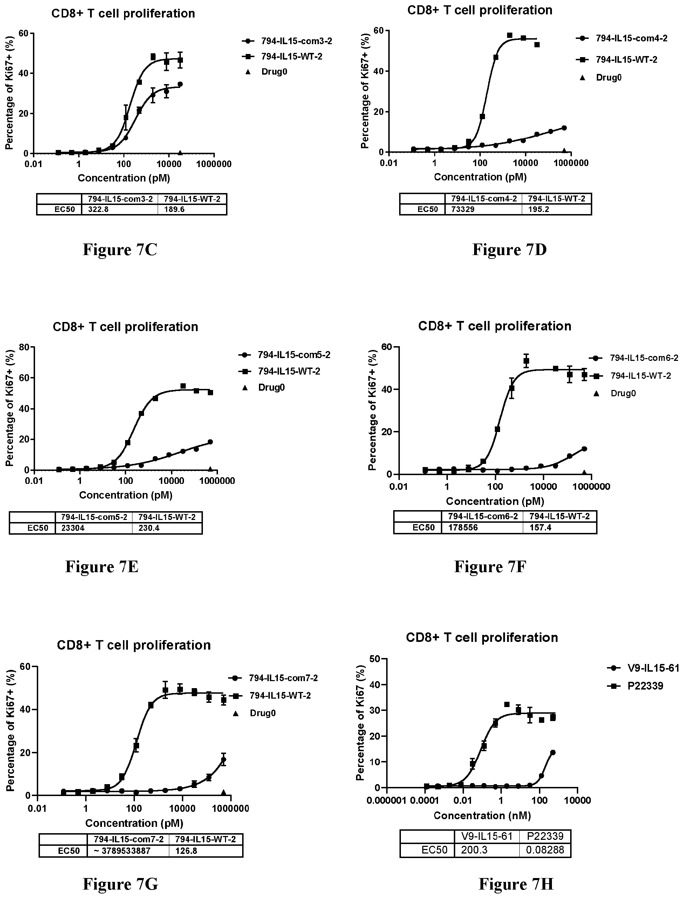

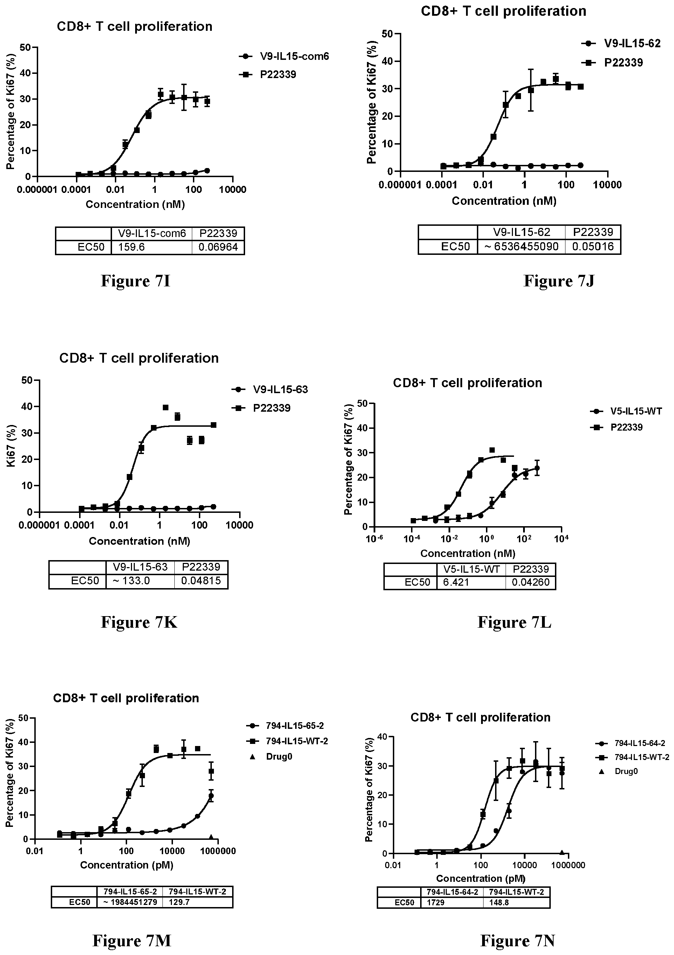

Figures 7A-7N show IL-15 mutant induces CD8+ T cell proliferation:
   Figure 7A: the structure of the fusion protein is as shown in Figure 5A, and Tecentriq is fused to IL-15Rαsushi, and the IL15 mutant is non-covalently linked to the IL-15Rαsushi without linker ligation;
   Figures 7B-7G and 7M-7N: the structure of the fusion protein is as shown in Figure 5B, and the self-developed PD-L1 monoclonal antibody is fused to IL-15Rαsushi and IL15 mutant, and the IL15 mutant is linked with IL-15Rαsushi by linker for tandem expression; DrugO represents a negative control in which no protein is added to the reaction system;
   Figures 7H-7K: the structure of the fusion protein is as shown in Figure 5D, and IL-15Rαsushi is first linked to IL-15 wild-type or mutant by linker, and the IL-15 wild-type or mutant and Fc are then fused and assembled in tandem by linker for expression;
   Figure 7L: the fusion protein is as shown in Figure 5C, and IL-15 wild-type or mutant is first linked to IL-15Rαsushi by linker, and the IL-15Rαsushi and Fc are then fused and assembled in tandem by linker for expression.

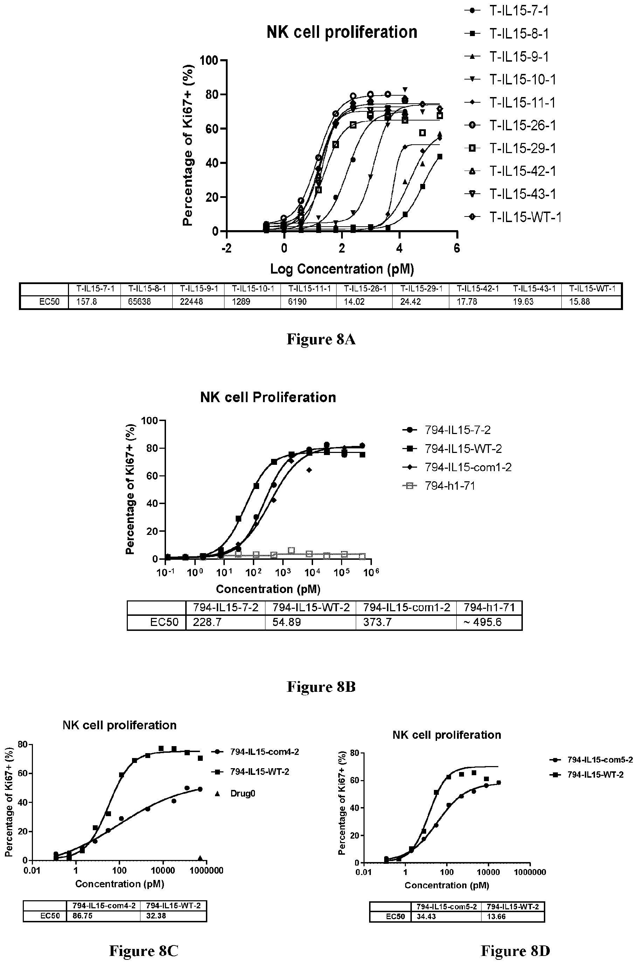

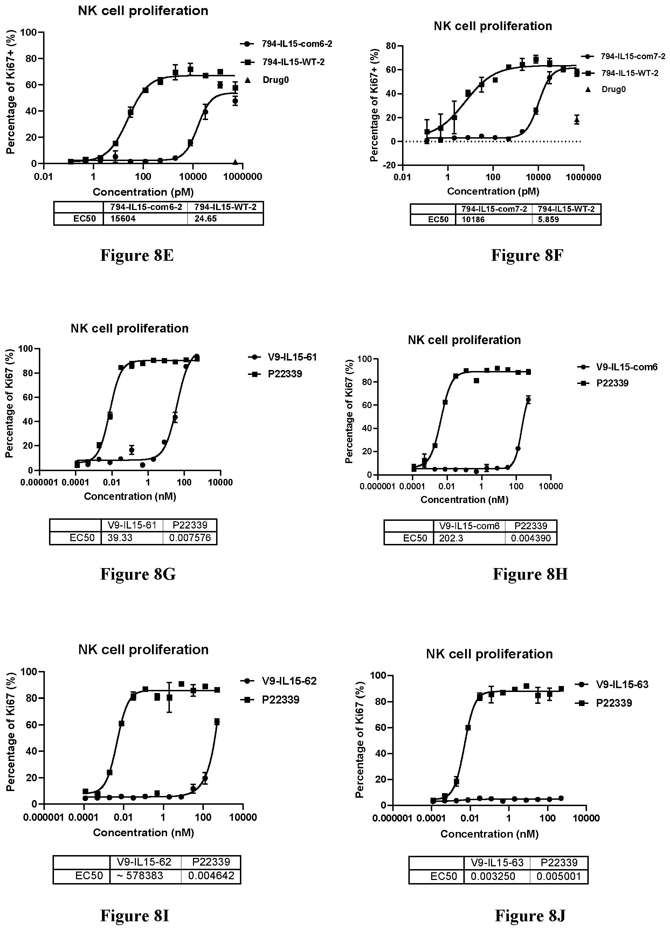

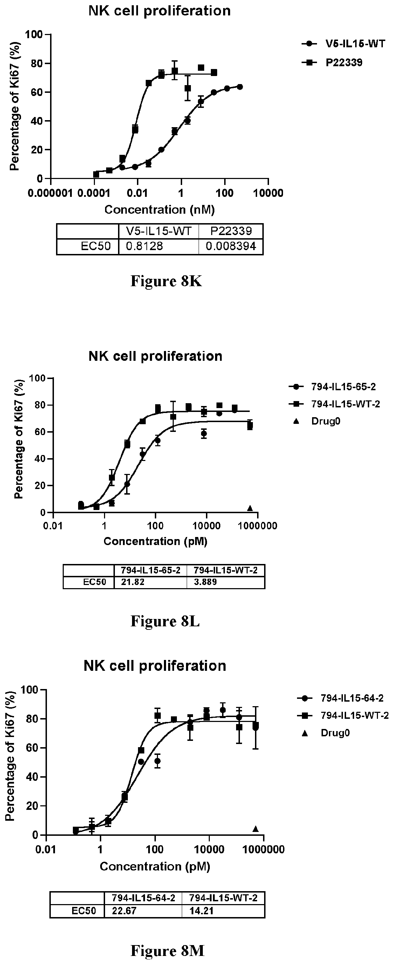

Figures 8A-8M show IL-15 mutant Induces NK cell proliferation:
   Figure 8A: the structure of the fusion protein is as shown in Figure 5A, and Tecentriq is fused to IL-15Rαsushi, and the IL15 mutant is non-covalently linked to the IL-15Rαsushi without linker ligation;
   Figures 8B-8F and 8L-8M: the structure of the fusion protein is as shown in Figure 5B, and the self-developed PD-L1 monoclonal antibody is fused to IL-15Rαsushi and IL15 mutant, and the IL15 mutant is linked with IL-15Rαsushi by linker for tandem expression; DrugO represents a negative control in which no protein is added to the reaction system.
   Figures 8G-8J: the structure of the fusion protein is as shown in Figure 5D, and IL-15Rαsushi is first linked to IL-15 wild-type or mutant by linker, and the IL-15 wild-type or mutant and Fc are then fused and assembled in tandem by linker for expression.
   Figure 8K: the fusion protein is as shown in Figure 5C, and IL-15 wild-type or mutant is first linked to IL-15Rαsushi by linker, and the IL-15Rαsushi and Fc are then fused and assembled in tandem by linker for expression.

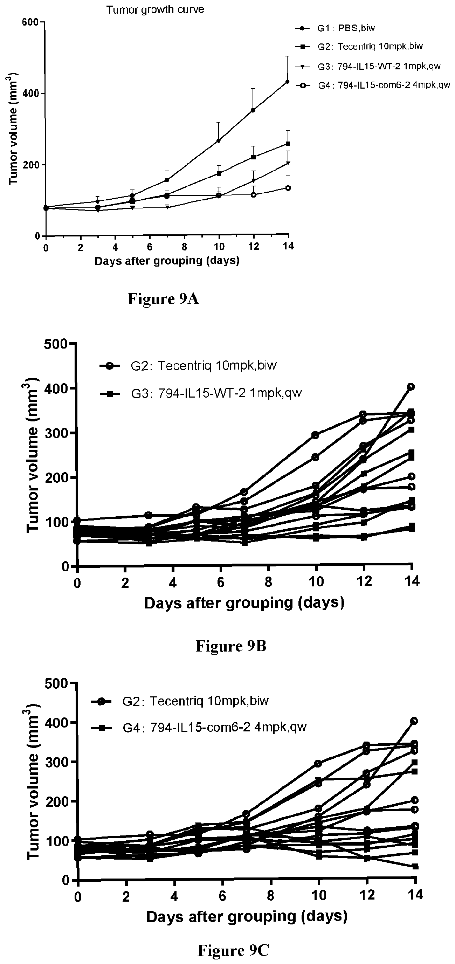

Figures 9A-9C: In vivo pharmaceutical efficacy in hPBMC-A375 mice. The fusion protein is as shown in Figure 5B.

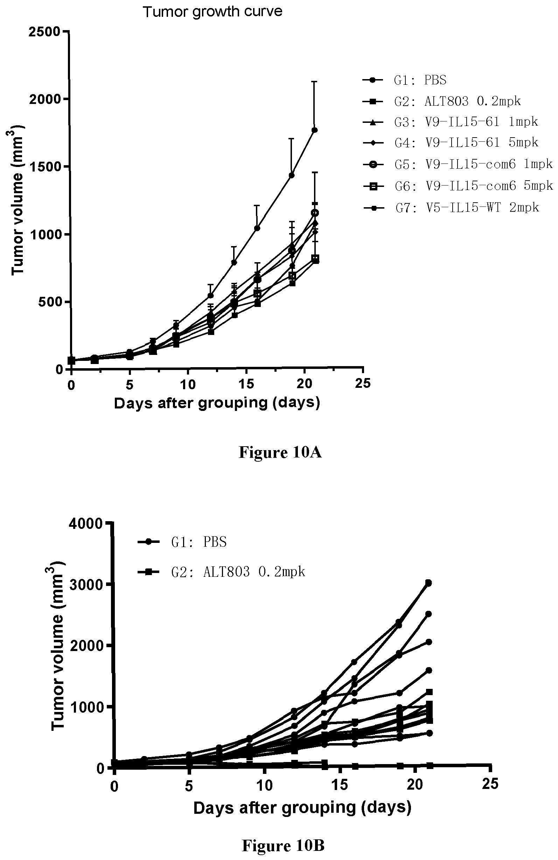

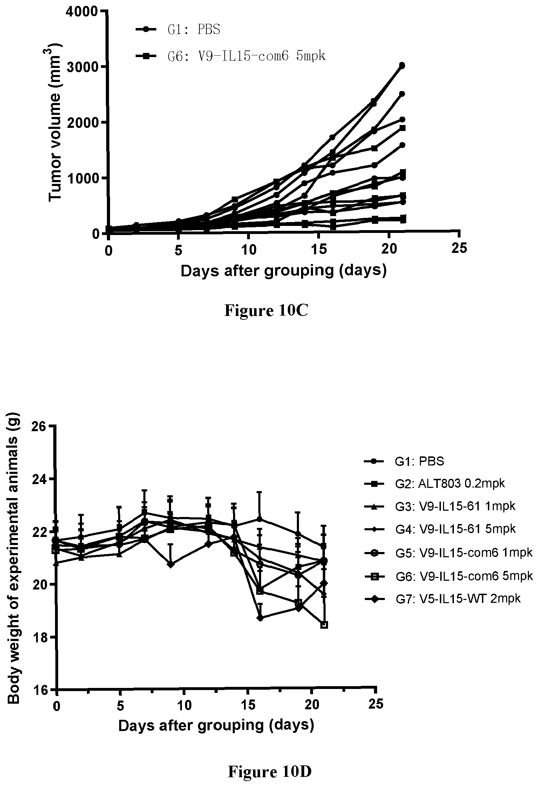

Figures 10A-10C: In vivo pharmaceutical efficacy in hPBMC-A375 mice. The fusion protein is as shown in Figure 5C or SD.
Figure 10D: changes of body weight in hPBMC-A375 mice after administration.

### DETAILED DESCRIPTION OF THE EMBODIMENTS

The present disclosure will be further described below with reference to specific examples, and the advantages and characteristics of the present disclosure will become clearer with the description. If the specific conditions are not indicated in the examples, it is carried out according to the conventional conditions or the conditions recommended by the manufacturer. The reagents or instruments used without the manufacturer's indication are conventional products that can be purchased from the market.

The examples of the present disclosure are only exemplary, and do not constitute any limitation on the scope of the present disclosure. It should be understood by those skilled in the art that the details and forms of the technical solutions of the present disclosure can be modified or replaced without departing from the spirit and scope of the present disclosure, and these modifications and replacements all fall within the protection scope of the present disclosure.

### Example 1. Antibody humanization

Firstly, antibody was humanized by employing the classical "CDRs grafting", *i.e.,* the human antibodies with the highest sequence homology were selected to provide the antibody framework regions (FRs), and the antigen-binding fragment complementarity determining regions (CDRs) of the target antibody based on the Kabat nomenclature were grafted into the former to form a humanized antibody. Secondly, in order to better maintain activity and affinity of the antibody, MOE software was used for modeling analysis based on the antibody structure: 1). amino acid residues located at the VH-VL interface, closed to CDRs or direct interacting with CDRs in the framework region of the antibody, were selected for reverse mutation, and such amino acid residues were mostly more important to maintain the conformation of the CDRs; 2). considering the immunogenicity, amino acids embedded in the protein were selected as far as possible for reverse mutation; 3). considering the stability and expression level of the antibody, molecular energy reduction mutations were preferred. Humanized antibodies with equivalent or better affinity, antibody characterization, and activity function to murine PD-L1 antibodies were screened out by detecting the binding affinity of humanized antibodies containing different mutations with human PD-L1 and cells expressing PD-L1 on the surface.

Among them, the amino acid sequence information of the heavy chain and light chain variable regions of the preferred candidate antibody molecule (794-h1-71) after humanization of the murine PD-L1 antibody (PDL1-794) was shown in Table 1 below.

**Table 1. Specific sequence information of heavy chain and light chain variable regions of murine and humanized anti-PD-L1 antibodies**

| **Antibody No.** | **Sequence No.** | **Sequence of variable region of heavy chain (VH)** |
|---|---|---|
| PDL1-794 | SEQ ID NO.97 | EVQLQESGPSLVKPSQTLSLTCSVTGDSITSGYWNWIRKFPGNKLE YMGYISYSGSTYYNPFLKSRISITRDTSKNQYYLQLNSVTTEDTAT YYCAKMGDWLAWFAYWGQGTTVTVSS |
| 794-h1-71 | SEQ ID NO.99 | QVQLQQSGPGLVKPSQTLSLTCAVSGDSITSGYWNWIRKFPSRGL EYMGYISYSGSTYYNPFLKSRISINRDTSKNQYYLQLNSVTPEDTA VYYCAKMGDWLAWFAYWGQGTLVTVSS |

| **Antibody No.** | **Sequence No.** | **Sequence of variable region of light chain (VL)** |
|---|---|---|
| PDL1-794 | SEQ ID NO.98 | EIVMTQSPSSLAVSVGEKVTLSCKSSQSLLYSSNQKNSLAWYQQK PGQSPKLLIYWASTRESGVPDRFTGSGSGTDFTLTISSVKAEDLAV YYCQQYYGYPYTFGGGTKLEIK |
| 794-h1-71 | SEQ ID NO.100 | EIVMTQSPPTLSLSPGERVTLSCKSSQSLLYSSNQKNSLAWYQQKP GQAPRLLIYWASTRESGIPARFSGSGSGTDFTLTISSLQPEDFAVYY CQQYYGYPYTFGQGTKLEIK |

### Example 2 Expression and purification of humanized antibody

### 2.1 Expression of Humanized Antibody

On the day prior to transfection, ExpiCHO-S cells (Thermo fisher, A29127) were seeded in fresh ExpiCHO Expression medium (Invitrogen, A29100-01) at a density of 2.5 × 10⁶ to 4 × 10⁶ cells/mL and cultured overnight on a shaker. On the day of transfection, ExpiCHO-S cell suspension cultured overnight was counted, and the cell viability was > 95% and the cell density was between 7 × 10⁶ and 10 × 10⁶ viable cells/mL. The desired cell suspension was diluted to a density of 6 × 10⁶ cells/mL with ExpiCHO Expression medium (Invitrogen, A29100-01) and placed on a shaker for further use. The prepared plasmid for humanized antibody expression was diluted in the medium, mixed well by gently shaking the centrifuge tube, added with the OptiPRO^{™} SFM-DNA diluent (Invitrogen, 12309-019), and allowed to stand at room temperature for 1 to 5 min after mixing well by gently shaking the centrifuge tube. The above plasmid complex was slowly dropped into the cell suspension to be transfected, and the flask was shaken during the dropwise addition. After transfection, cells were cultured overnight on a shaker. Cells on the first day post-transfection were supplemented with 0.6% of the cell volume of ExpiFectamine^{™} CHO Enhancer (Invitrogen, A29129) and 16% of the cell volume of ExpiCHO^{™} Feed (Invitrogen, A29129), and the flask was shaken gently during the addition, and the cells were transferred to the shaker for culturing for 4 days. On day 5 post-transfection, the transfected cells were supplemented with 16% of the cell volume of ExpiCHO^{™} Feed (Invitrogen, A29129), and the flask was gently shaken during addition. On day 12 post-transfection, 9,000 g of culture medium was taken and centrifuged for 10 min before harvesting the supernatant.

### 2.2 Purification of Humanized Antibody

The supernatant of the cell culture collected in Example 2.1 was centrifuged at high speed and passed through 0.45 µm + 0.22 µm filter membranes. The first step of purification was performed by using affinity chromatography. The chromatography media was protein A filler Mbaselect Sure (GE, 17543803) interacting with Fc, and the equilibration buffer was PBS (2.5 g/L Na₂HPO₄·12H₂O, 0.408 g/L NaH₂PO₄, 8.76 g/L NaCl, pH7.2). After balancing the column with 4 times column volume of PBS, the cell supernatant was loaded and combined with the column, and the flow rate was controlled so that the retention time of the sample on the column ≥ 5 min. After loading the sample, the column was washed with PBS (pH7.2) until the A280 UV absorbance dropped to the baseline. The column was then washed with 2 times column volume of 20 mM PB + 1 M NaCl (pH 6.0). The column was then washed with PBS (pH 7.2) until the A280 UV absorbance and conductivity reached baseline. Finally, the column was washed with elution buffer of 20 mM citric acid (pH 3.4), and the elution peak was collected based on the A280 UV absorption peak. The collected elution samples were neutralized to neutrality with 1 M Tris- HCl (pH 9.0).

### Example 3. Binding of antibody to human and cynomolgus monkey PD-L1 recombinant protein analyzed by KD assay

Biacore T200 (GE Healthcare) was used to determine the binding affinity of PD-L1 antibody to human and cynomolgus monkey PD-L1-His protein. Anti-human IgG Fc (Genway, Cat. GWB-20A705) was immobilized on a CMS chip (GE Healthcare, Cat. BR-1005-30) at 25°C. Anti-human IgG Fc was diluted to 20 µg/mL with acetate, pH 5.0 (GE Healthcare, BR-1003-51). A amine-based immobilization method was used for immobilization. Alternatively, commercial Protein A (GE Healthcare, Cat. 29127556) chip could be used for detection. The affinity of the antibody to the antigen was determined by using multi-cycle kinetics at 25°C. In each cycle, the antibody to be tested was first captured on a fixed CMS chip, and then recombinant human PD-L1-His protein (Novoprotein, Cat. 315) and cynomolgus monkey PD-L1-His protein (Sino Biological, Cat. 90251-C08H) were injected, and finally Glycine pH1.5 (HUSHI, Cat. 62011516) was used for regeneration. The mobile phase was HBS-EP + Buffer (GE Healthcare, Cat .BR-1006-69) with a flow rate of 30 µL/min and a binding time of 300 seconds. The flow rate and time for regeneration was 30 µL/min and 30 seconds. The experimental data were analyzed using Biacore T200 Evaluation Software (version 3.0) with a 1:1 binding model to fit the equilibrium dissociation constant (KD) of the antibody and antigen, and determine the association rate constant (ka) and dissociation rate constant (kd).

It can be seen from the results that the tested PD-L1 antibody shows an nM level or higher affinity for binding human PD-L1 recombinant protein and cynomolgus PD-L1 recombinant protein, as shown in Table 2 below.

**Table 2. Results of binding affinity (KD) of humanized PD-L1 antibody by Biacore**

| **Antibody No.** | **Human PD-L1(M)** | **Cynomolgus monkey PD-L1(M)** |
|---|---|---|
| 794-h1-71 | 1.793E-09 | 9.372E-10 |

### Example 4. Blocking of the interaction between PD-L1 and PD-1 by antibody analyzed by IC50 assay

The IC50 of anti-PD-L1 antibody blocking the binding of PD-L1 protein to PD-1 protein was determined by competitive ELISA. Human PD-L1 recombinant protein (Sino Biological, Cat.10084-H05H) was diluted with carbonate buffer and added to 96-well ELISA plate at a final concentration of 1 µg/ml. After blocking with 3% BSA in PBS and co-incubating with gradient diluted anti-PD-L1 antibody (40 nM to 0.02 nM) and human PD-1-His recombinant protein (Sino Biological, Cat.10377-H08H), HRP-labeled anti-His-tag antibody (MBL, Cat.D291-7) and TMB (Thermo, Cat.34029) were added for color development, and OD values (dual wavelength 450 nm - 630 nm) were read after termination with 1 M sulfuric acid. The competitive binding curve of the tested antibody could be drawn by matching the antibody concentration to the OD value, and the IC50 value could be calculated. **Figure 1** shows the competitive binding curve of anti-PD-L1 antibody to human PD-L1 recombinant protein. The result shows that the tested antibody (794-h1-71) could effectively block the interaction between human PD-L1 protein and human PD-1 protein, with an IC50 of 0.8488 nM, and the IC50 of positive control, Tecentriq (Genetech, lot: H0172) is 0.8486 nM.

### Example 5. EC50 of PD-L1 antibody binding to PD-L1 on cell surface detected by FACS assay

The antibody to be detected with gradient concentrations (antibody concentrations: 10000 ng/ml - 0.1 ng/ml) were incubated with CHO-PD-L1 cells (Nanjing Yongshan Biotechnology Co., Ltd., 10⁵ cells/well) highly expressing PD-L1 on the cell surface for 30 min at 4°C. After the incubation, diluted (1:250) anti-human IgG PE fluorescent antibody (eBioscience, Cat. 12-4998-8) was added to above culture and incubated at 4°C for 30 min. The fluorescent antibody produced specifically bound to the Fc segment of the antibody to be detected. The ability of the antibody to be detected binding the PD-L1 protein highly expressed on the cell surface was analyzed through detecting the level of PE fluorescence intensity by FACS. The result of **Figure 2** shows that the EC50 of 794-h1-71 antibody is 38.44 ng/ml, which is similar to that of Avelumab (EC50 is about 72 ng/ml), the positive control in this experiment. The assay quantitatively confirms the dose-dependent binding ability of the 794-h1-71 antibody to the PD-L1 target on the cell surface. Mean fluorescence intensity fold (MFI fold) = MFI value of experimental group/MFI value of control group without adding drugs (antibodies).

### Example 6. Inhibition of binding and signal transduction of PD-1: PD-L1 by anti-PD-L1 antibody analyzed by PD-1/PD-L1-NFAT reporter gene

The antagonistic effect of PD-L1 antibody on PD-1/PD-L1 protein interaction and its signaling pathway was compared using Jurkat cell line (GenScript, Cat.00612) stably transfected with PD-1 and CHO cell line (GenScript, Cat. M00613) stably transfected with PD-L1. When the inhibitory signaling pathway was inhibited, the expression of NFAT-controlled luminescent reporter gene was enhanced and thus the luminescent signal value was increased. The blocking effect of antibodies on PD-L1 was reflected by the intensity (relative light units, RLU) of the luminescence readings.

CHO cell lines stably transfected with PD-L1 were seeded in 96-well white bottom plates at a density of 40,000 cells/well (100 µl/well) and cultured in the incubator overnight. The next day, the culture medium in the plate was discarded, and the cell lines stably transfected with PD-1 (16,000 cells/well) and PD-L1 antibody (gradient diluted, each dose is in triplicate) to be tested were added for co-incubation, with an incubation volume of 100 µl/well and an incubation time of 6 hours. When the incubation was completed, an equal volume (100 µl) of luminescence detection reagent was added into the plate, and the values were read. According to the detected values, a regression curve was made by performing 4-parameter analysis with Graphpad to obtain the EC50 value of each antibody. **Figure 3** shows that the EC50 of the antibody, 794-h1-71 (166.2 ng/ml) was similar to that of the positive control, avelumab (184.3 ng/ml). The assay quantitatively confirms that antibody (794-h1-71) shows a dose-dependent inhibitory ability against PD-1: PD-L1 interaction on cell surface, thereby dose-dependently enhancing the activity of reporter genes in Jurkat cells.

### Example 7. IFN-γ secreted by T cells in mixed lymphocyte reaction detected by ELISA

The T cell activity enhanced by PD-L1 monoclonal antibody was determined by mixed lymphocyte reaction (MLR). CD4⁺ monocytes were isolated from peripheral blood mononuclear cells (PBMCs) of healthy human donor 1 and induced to differentiate into dendritic cells (DCs) *in vitro* by using recombinant human granulocyte-macrophage colony-stimulating factor (GM-CSF, Peprotech, Cat. 300-03) and recombinant human interleukin 4 (rh IL-4, Peprotech, Cat. 200-04). LPS (Sigma, Cat: L4516) was added to stimulate DCs to mature on day 6 of culture. On day 7, DCs from donor 1 were co-cultured with CD4⁺ T cells enriched from PBMCs of healthy donor 2 (DCs: CD4⁺ of 1:10), the antibody to be tested, negative control antibody (anti-Hel, synthesized by Biointron) and positive control antibody, Avelumab (antibody concentration: 7 nM - 0.28 nM) for 4 days. 4 days later, cell culture supernatant was collected, and the content of IFN-γ in the supernatant was detected by ELISA. As shown in **Figure 4****,** compared with anti-Hel monoclonal antibody (negative control group), both 794-h1-71 and the positive control antibody, Avelumab, significantly enhance the ability of CD4⁺ T cells to secrete IFN-γ in MLR experiments, and with the decrease of the drug concentration of PD-L1 antibody, the activity of increasing IFN-γ secretion also decreases. This result indicates that the antibody (794-h1-71) can enhance T cell function in a dose-dependent manner (T-test, ^{∗}P < 0.05, ^{∗∗}P < 0.01, ^{∗∗∗}P < 0.001, ^{∗∗∗∗}P < 0.0001).

### Example 8. Construction of IL-15 Fusion Protein

MOE software was used to simulate critical amino acid sites for the interaction of human IL-15 with the corresponding receptor βγ chain, which were D8, V3, I6, and H105, respectively. According to simulation of MOE software, the following IL-15 mutant sequences were designed and synthesized, and the amino acid sequences are detailed in Table 3 and the coding nucleic acid sequences are detailed in Table 4.

The IL-15 fusion protein (antibody/Fc fusion construct/complex) was constructed in four modes:
(1) the structure as shown in Figure 5A, the IL-15 fusion protein is a homodimer containing two monomers; the monomer comprises an antibody heavy chain, an antibody light chain, an IL-15, and an IL-15Rα sushi; IL-15Rα sushi is fused to the Fc end of the antibody heavy chain and co-expressed with the light chain of monoclonal antibody and IL-15-WT (wild-type) or IL-15 mutant to make IL-15 non-covalently link to IL-15Rα sushi;
(2) the structure as shown in Figure 5B, the IL-15 fusion protein is a homodimer containing two monomers; the monomer comprises an antibody heavy chain, an antibody light chain, an IL-15, and an IL-15Rα sushi; the Fc end of the antibody heavy chain, IL-15Rα sushi and IL-15-WT or IL-15 mutant are sequentially fused in tandem for expression by linker and co-expressed with the antibody light chain;
(3) the structure as shown in Figure 5C (defined as V5), the IL-15 fusion protein is a homodimer containing two monomers; the monomer comprises a Fc, an IL-15, and an IL-15Rα sushi; the IL-15-WT or IL-15 mutant is linked to the IL15-Rαsushi by linker, and IL15-Rαsushi is then linked to the Fc by linker;
(4) the structure as shown in Figure 5D (defined as V9), the IL-15 fusion protein is a homodimer containing two monomers; the monomer comprises a Fc, an IL-15, and an IL-15Rαsushi; the IL15-Rαsushi is linked to the IL-15-WT or IL-15 mutant by linker, and the IL-15 is then linked to the Fc by linker.

The naming rules for PD-L1 antibody and IL-15 fusion protein are as follows:
"antibody acronym - IL-15 (wild-type/mutant) - fusion protein construction mode";
for example: "T-II,15-xx-1": "T", indicating Tecentriq; "IL15-xx", indicating IL15-WT or IL15 mutant; "1", indicating the structural mode shown in Figure 5A;
for example: "794- IL15-xx-2": "794", indicating 794-h1-71 monoclonal antibody; "IL15-xx", indicating IL15-WT or IL15 mutant; "2", indicating the structural mode shown in Figure 5B.

The various IL-15 fusion protein designs are shown in Table 5 and Table 6. According to the above four construction modes, the nucleic acid sequences encoding the fusion protein were constructed into the pTTS plasmid, respectively.

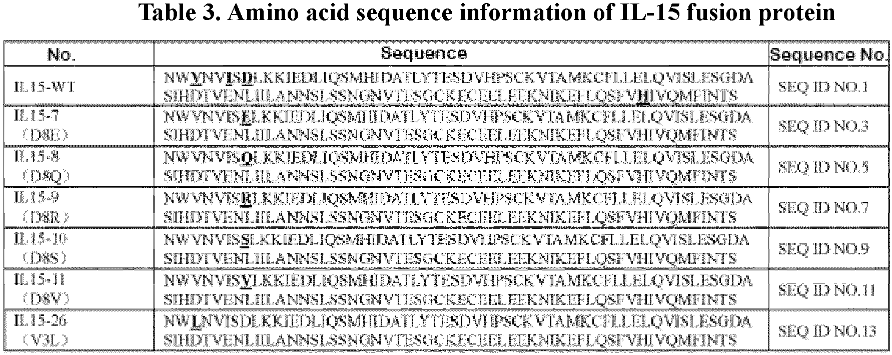

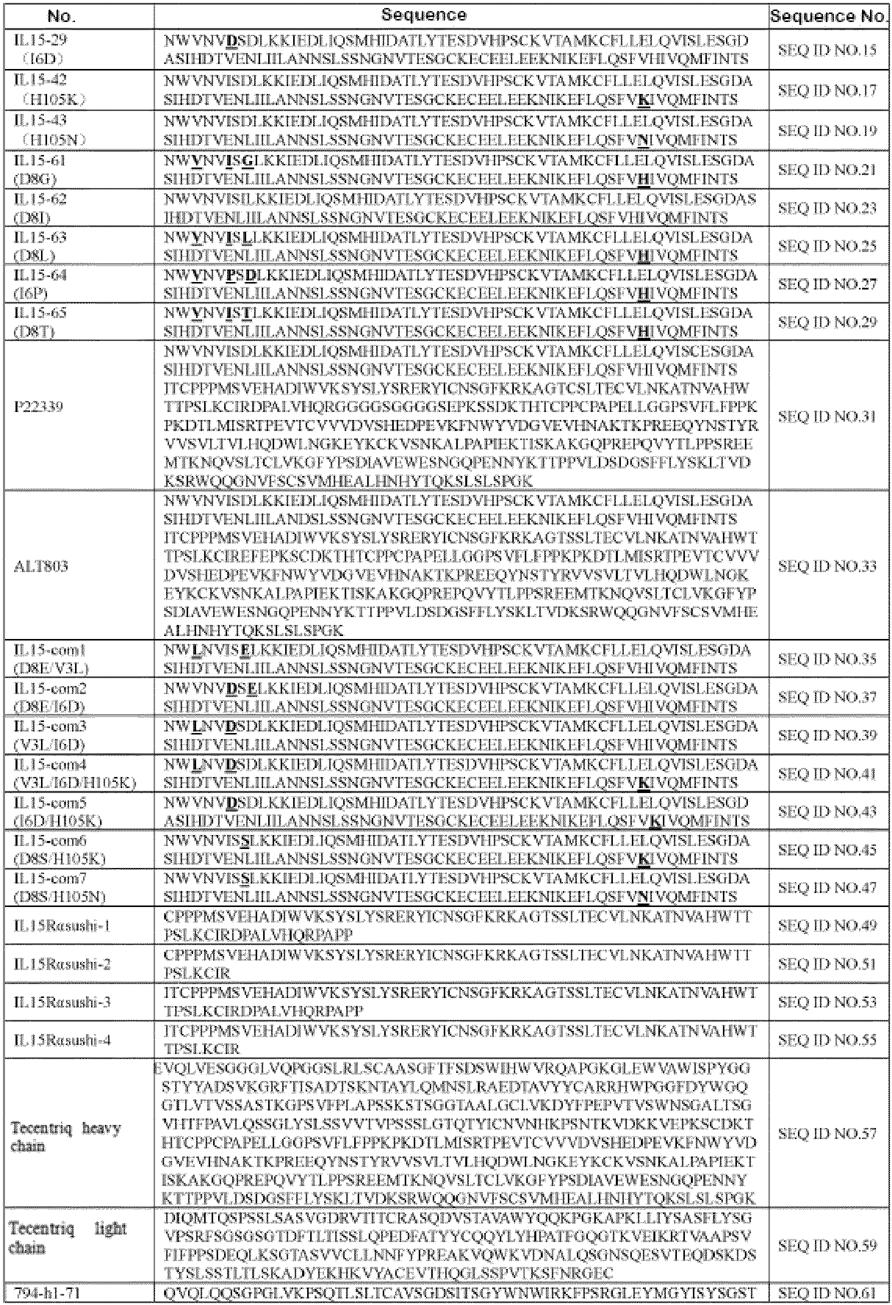

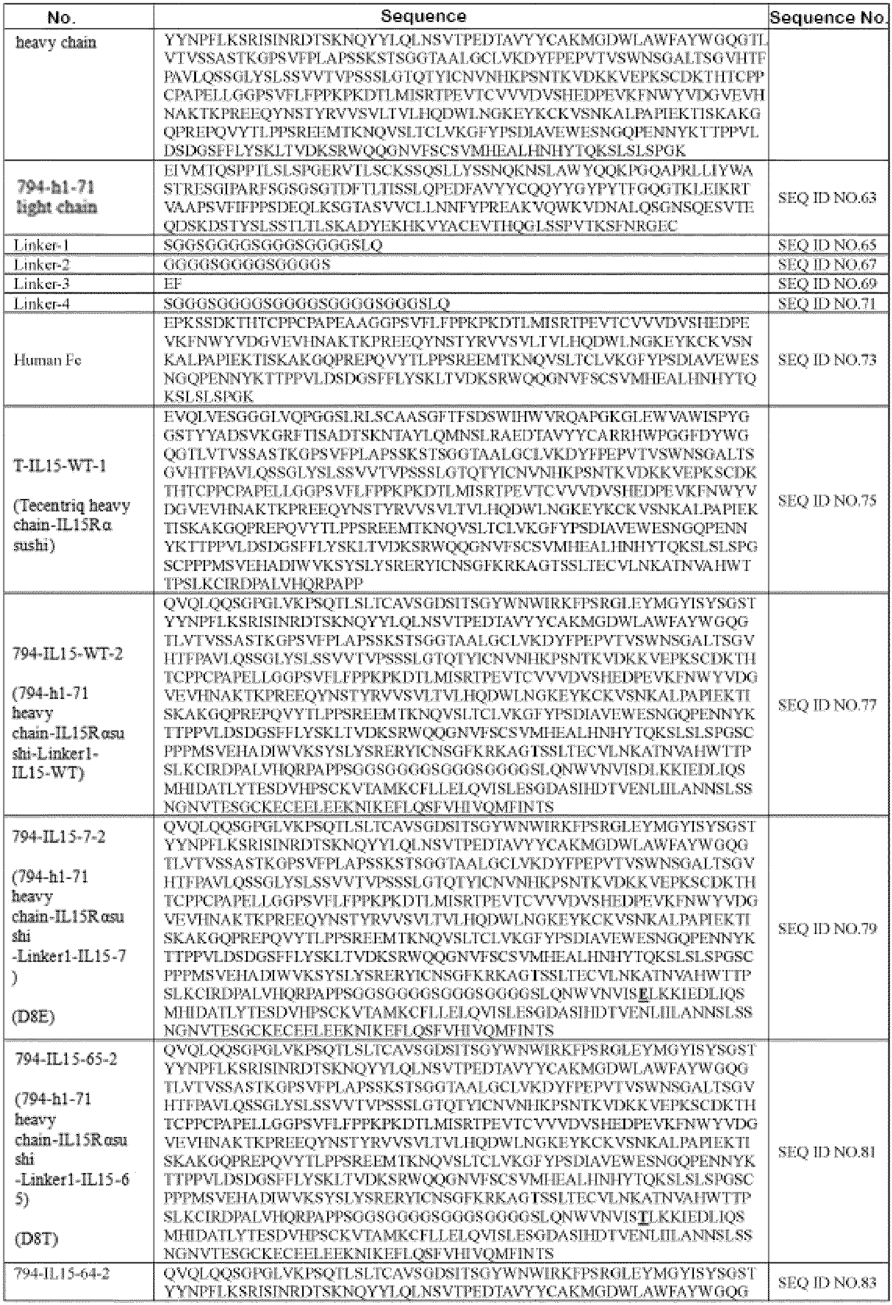

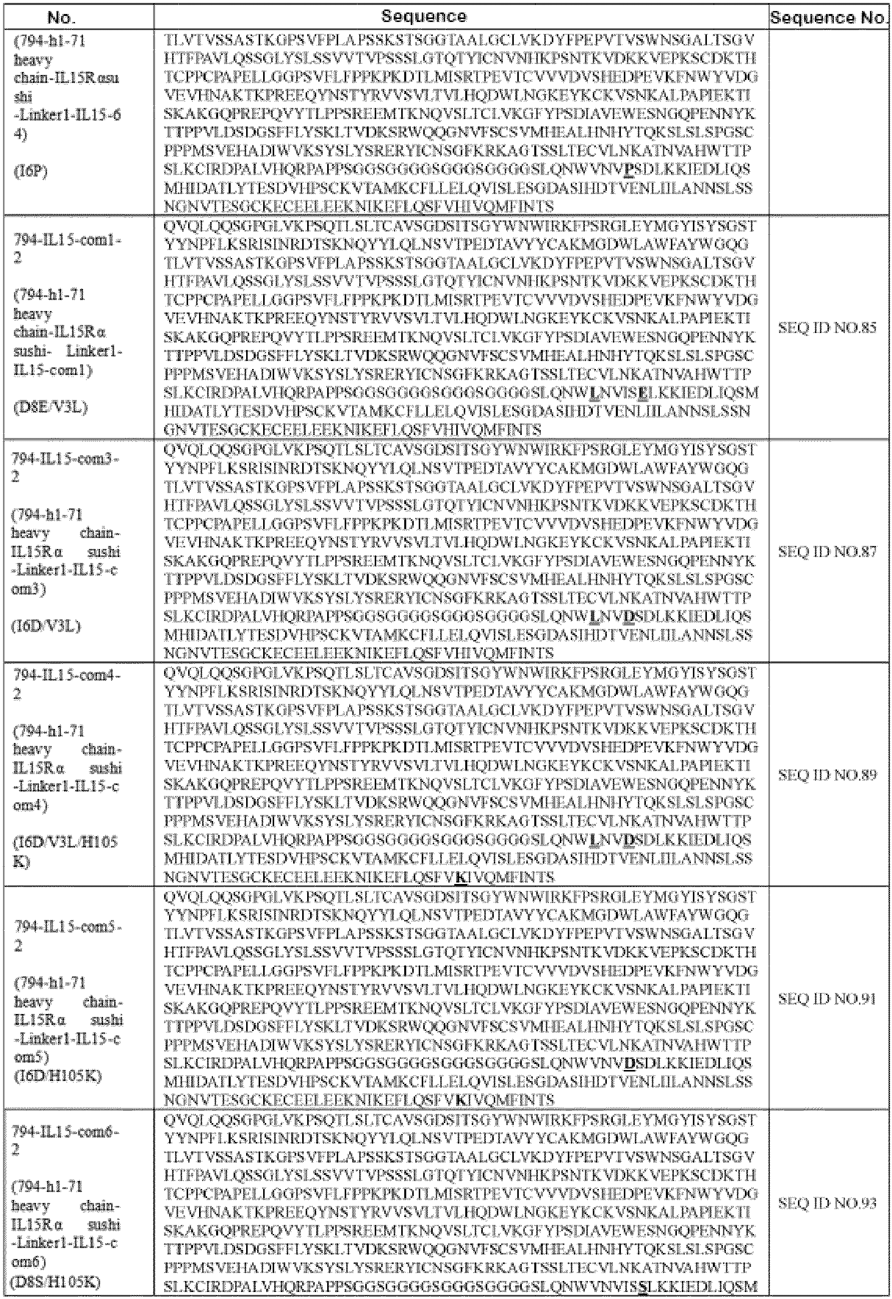

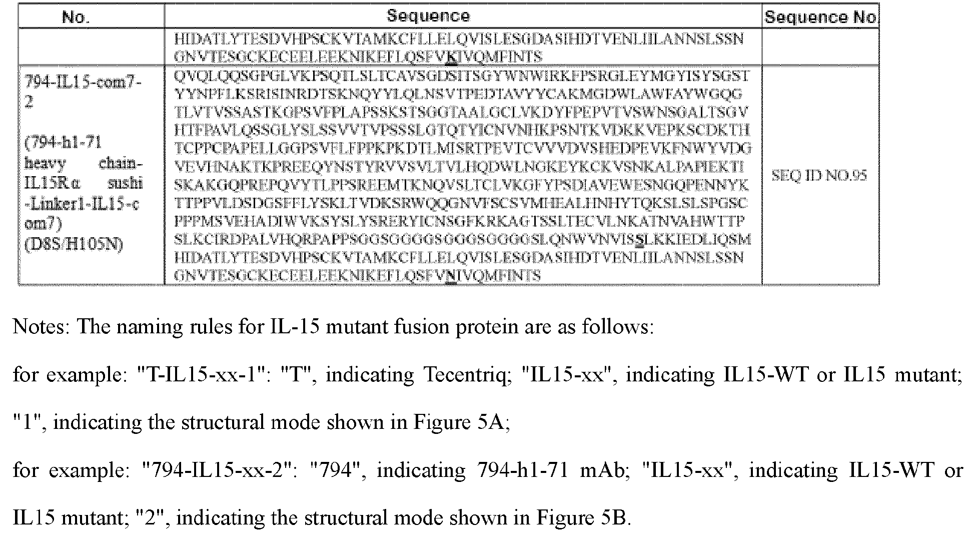

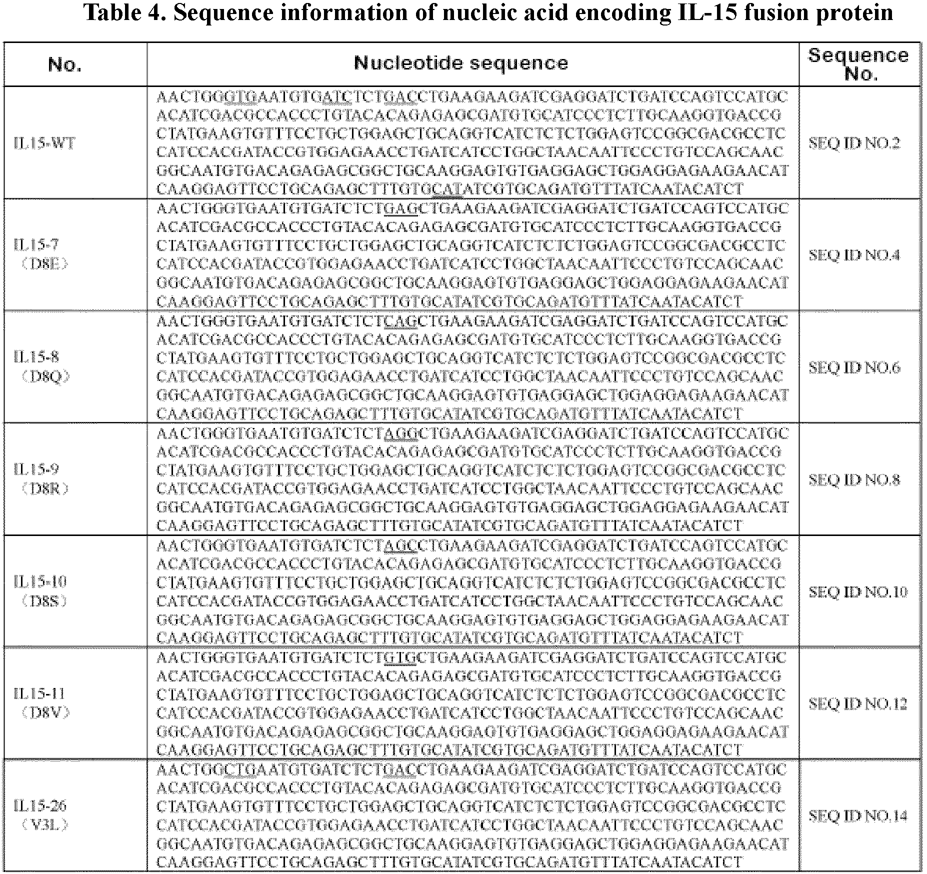

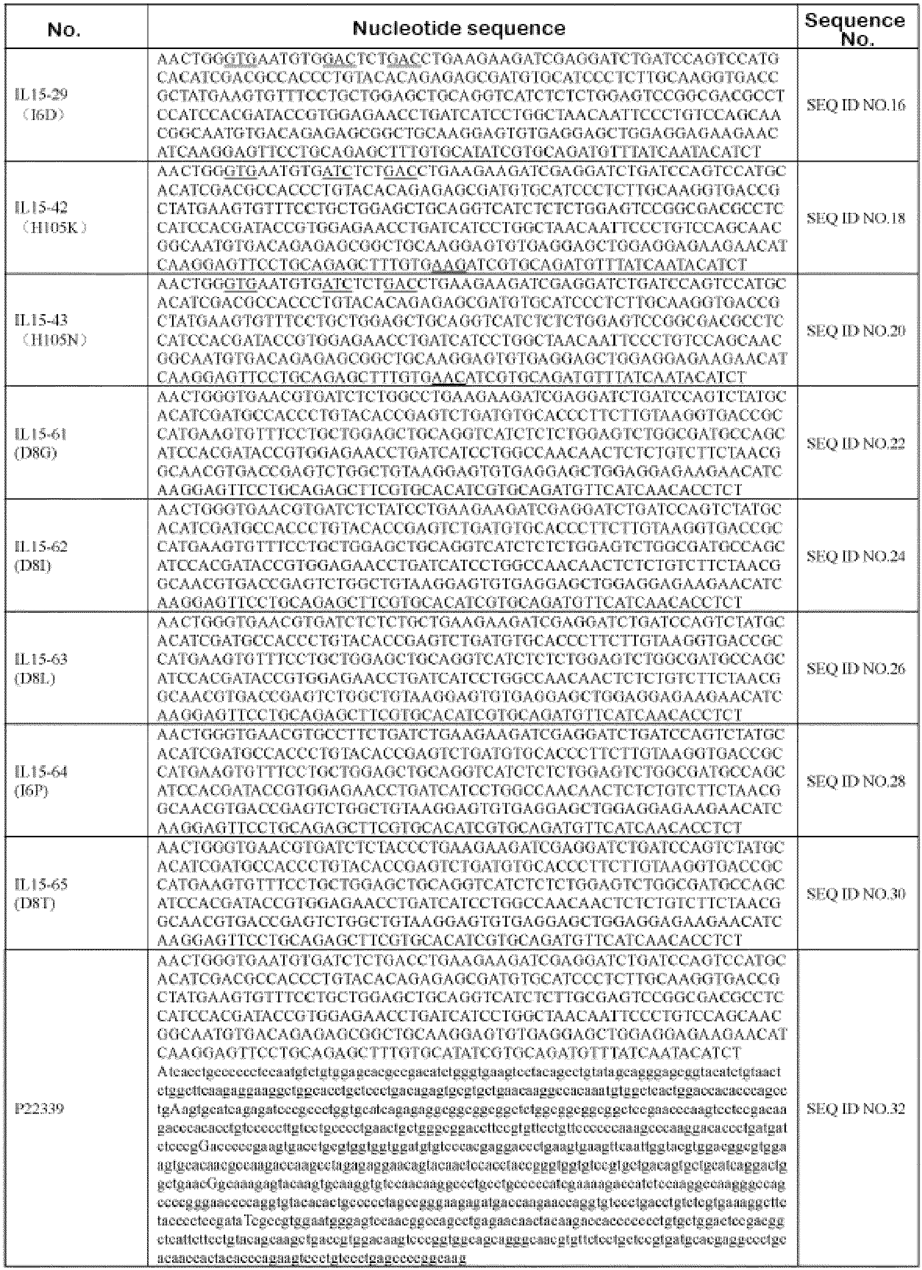

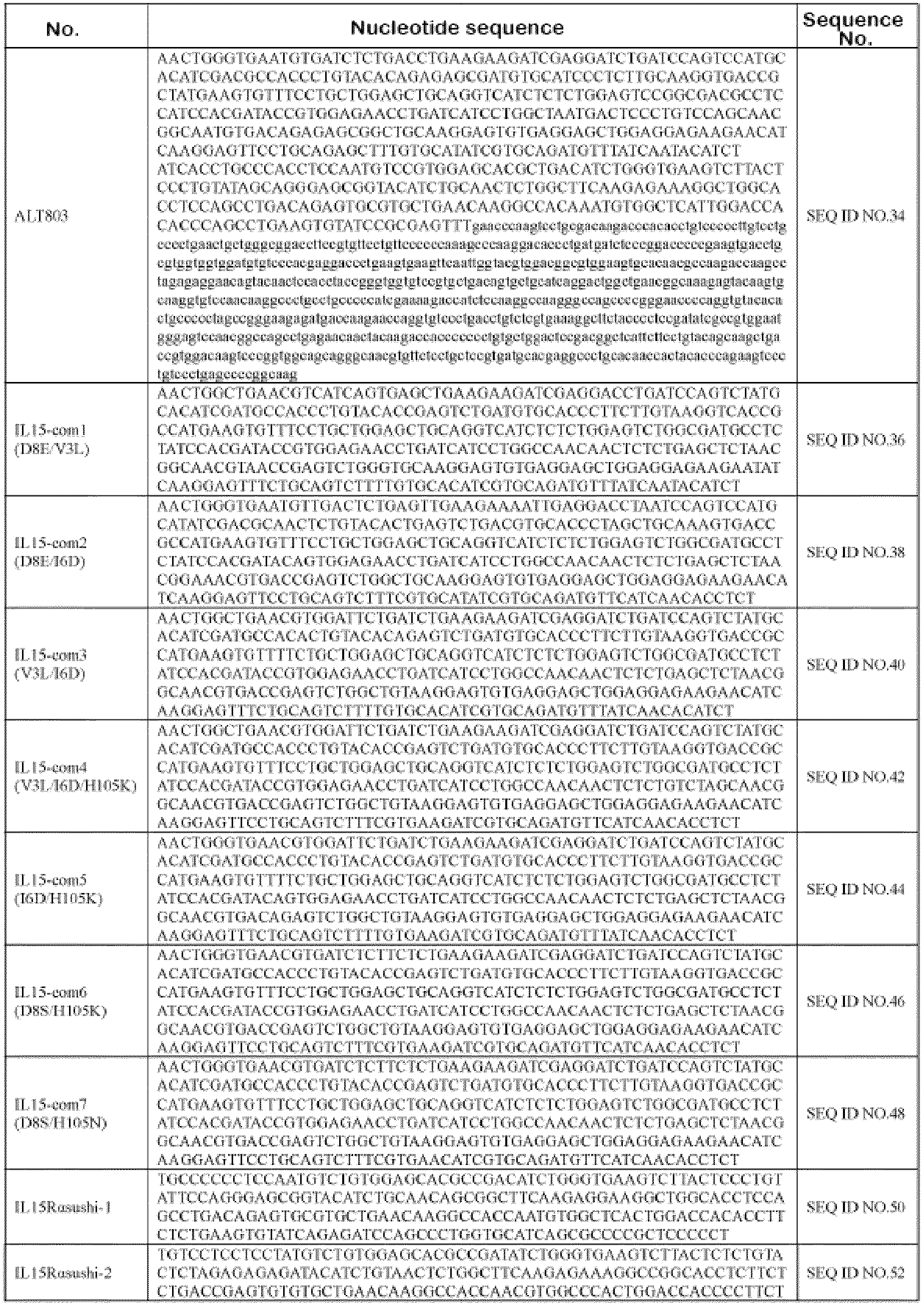

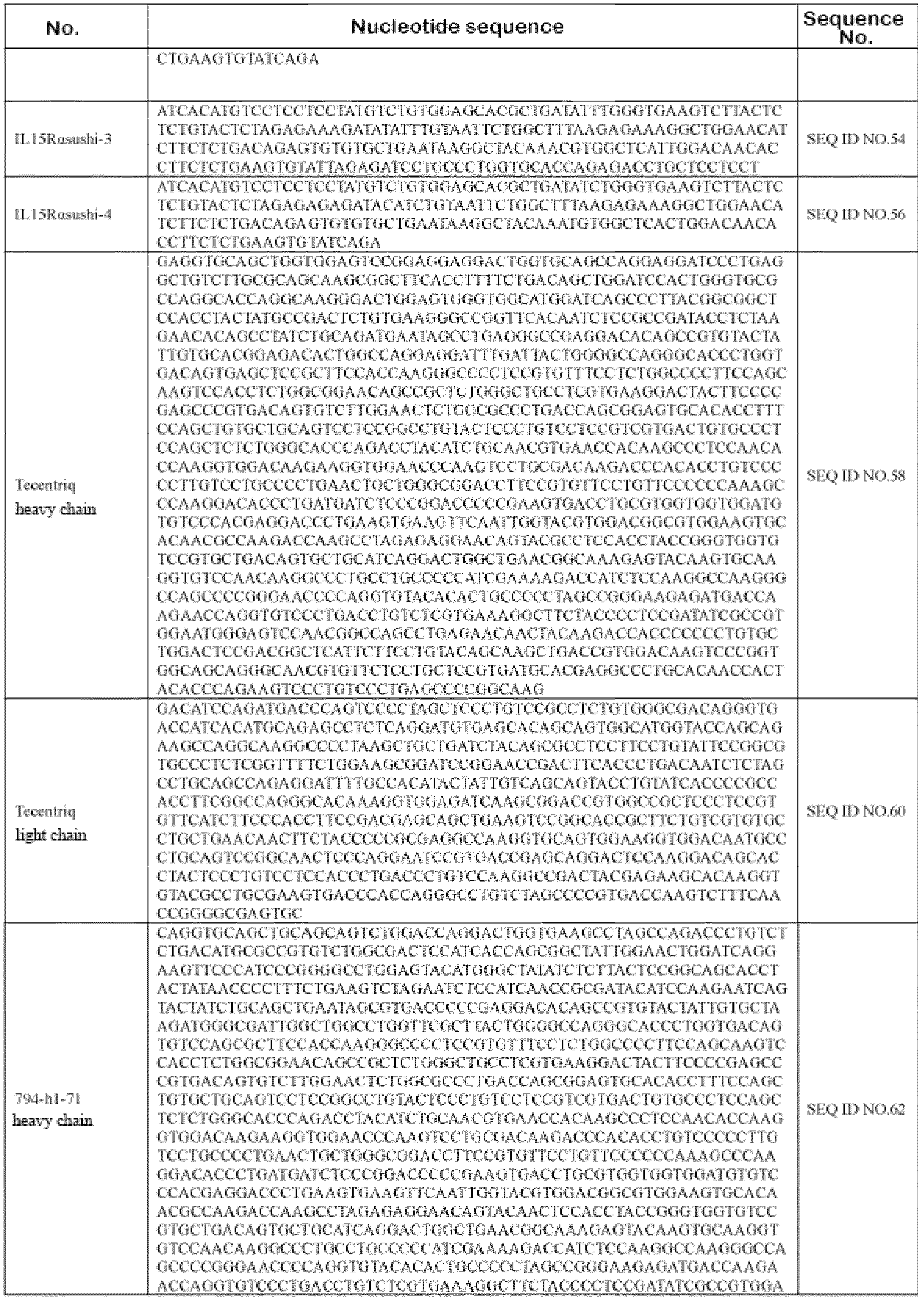

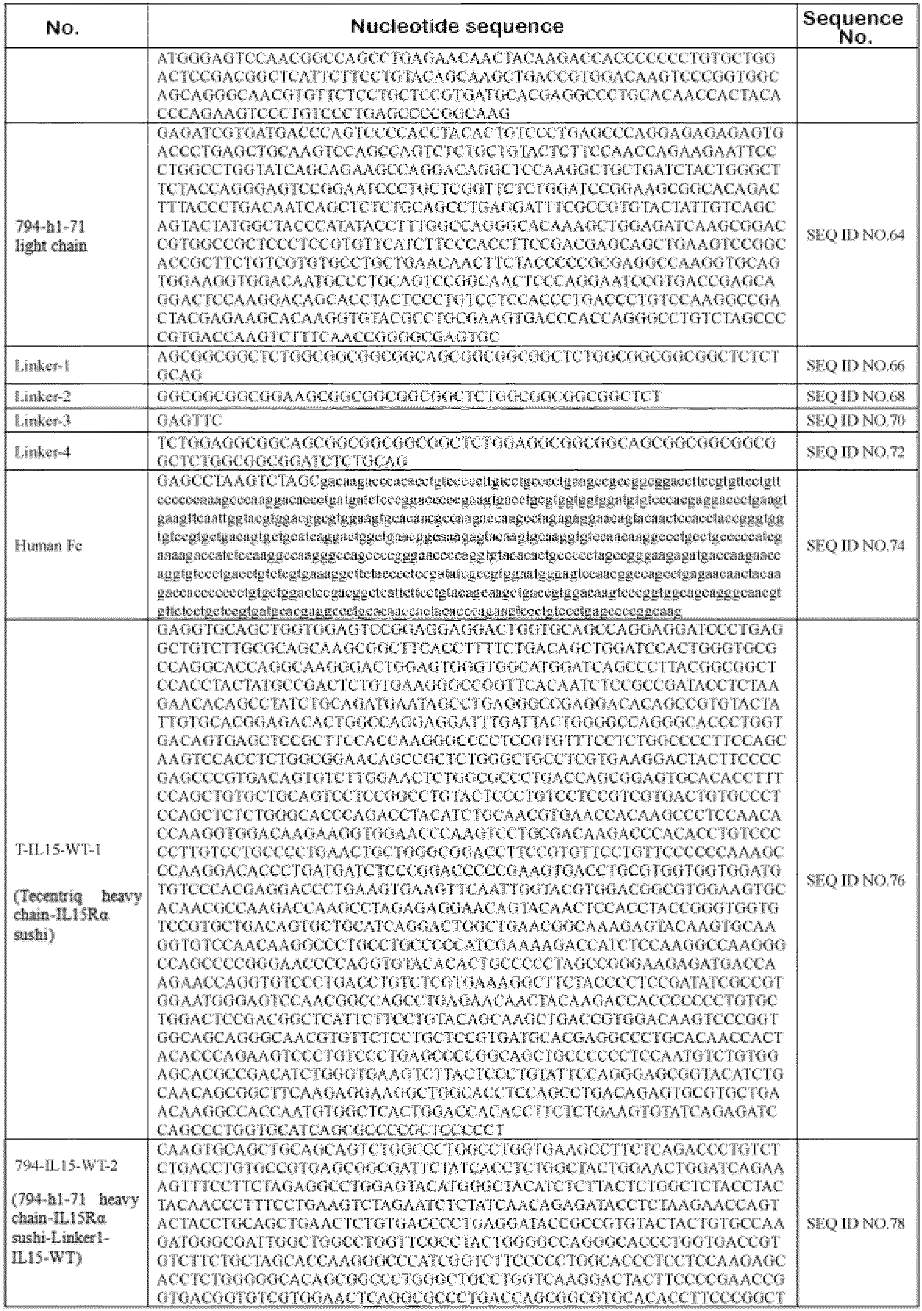

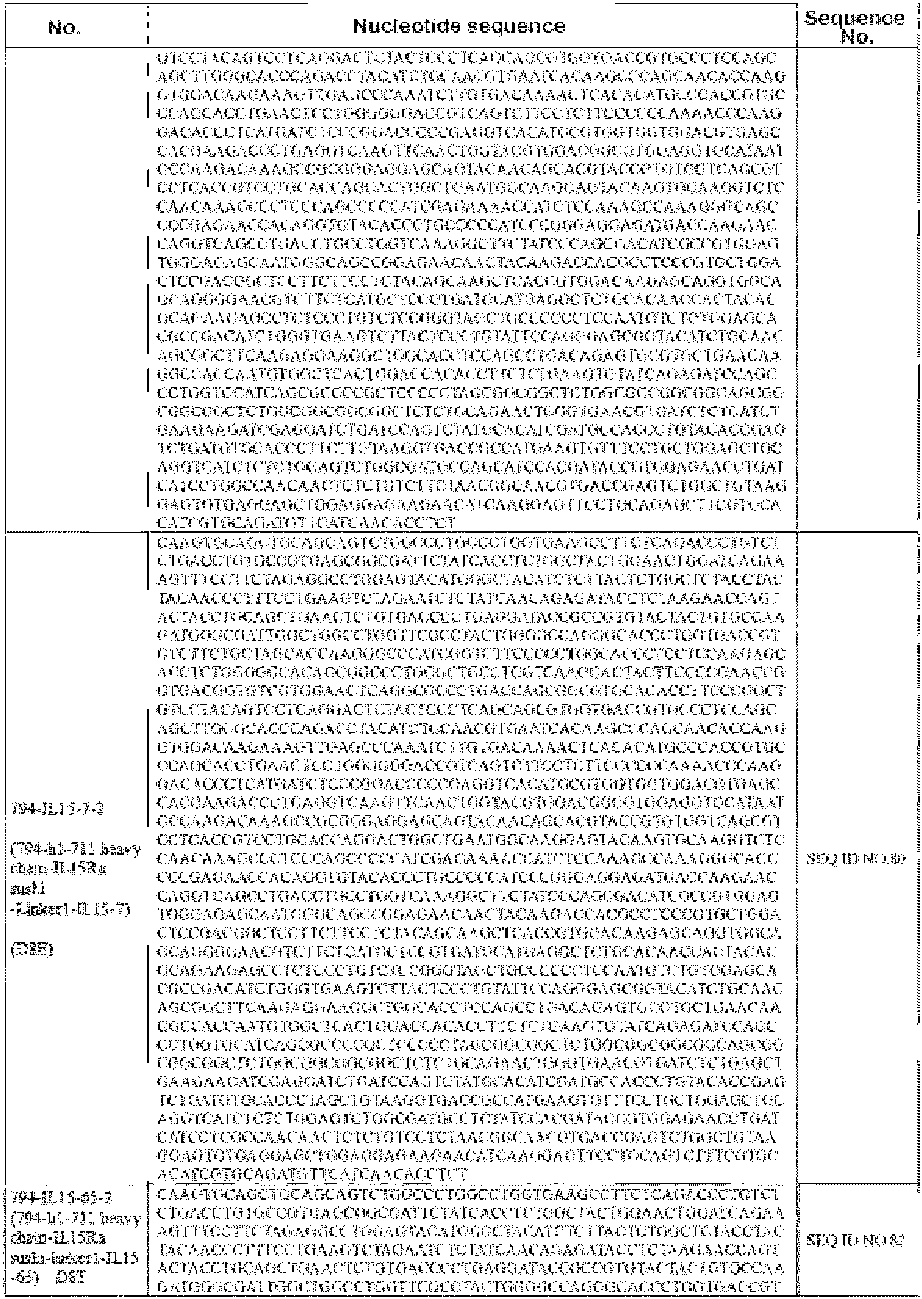

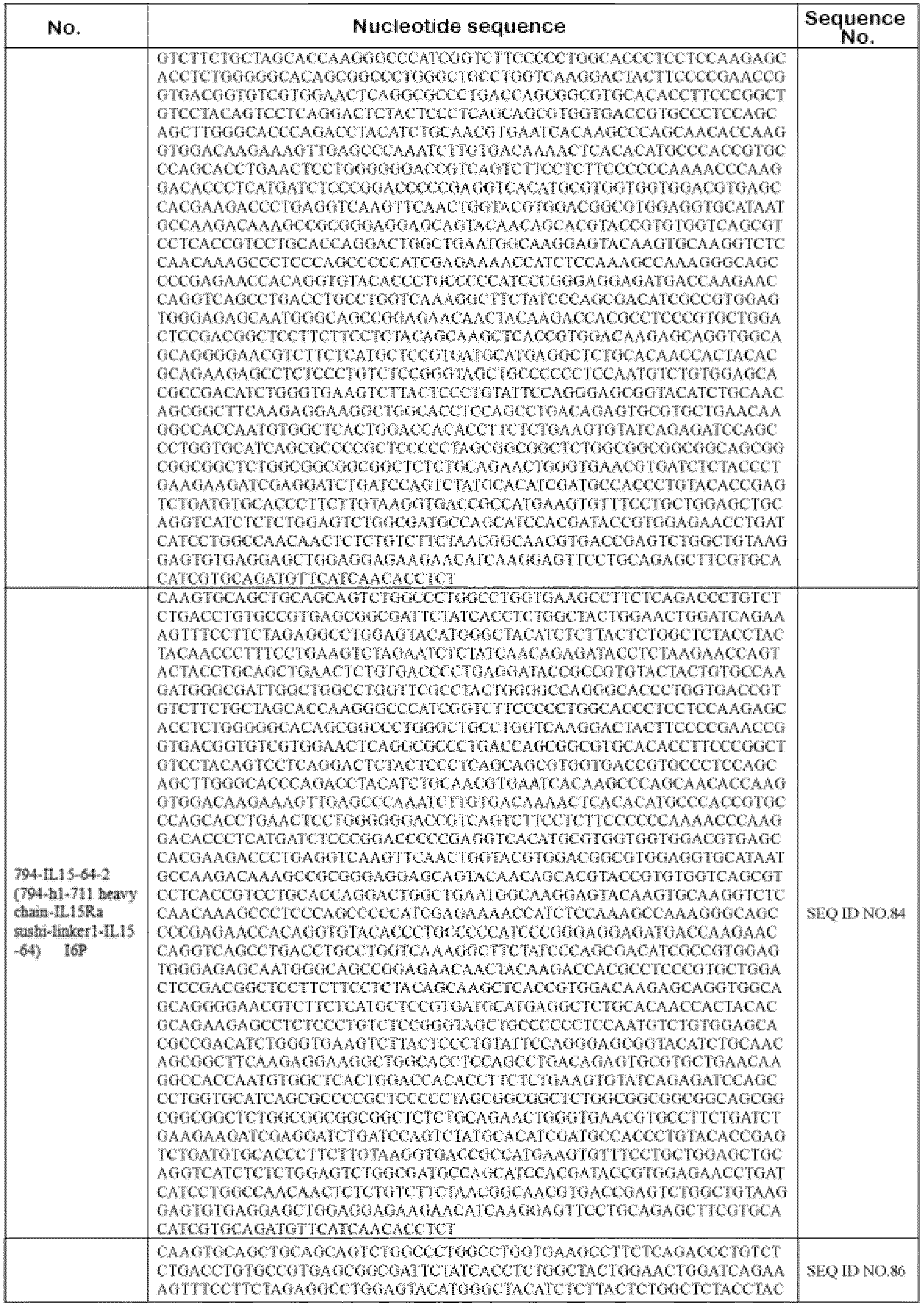

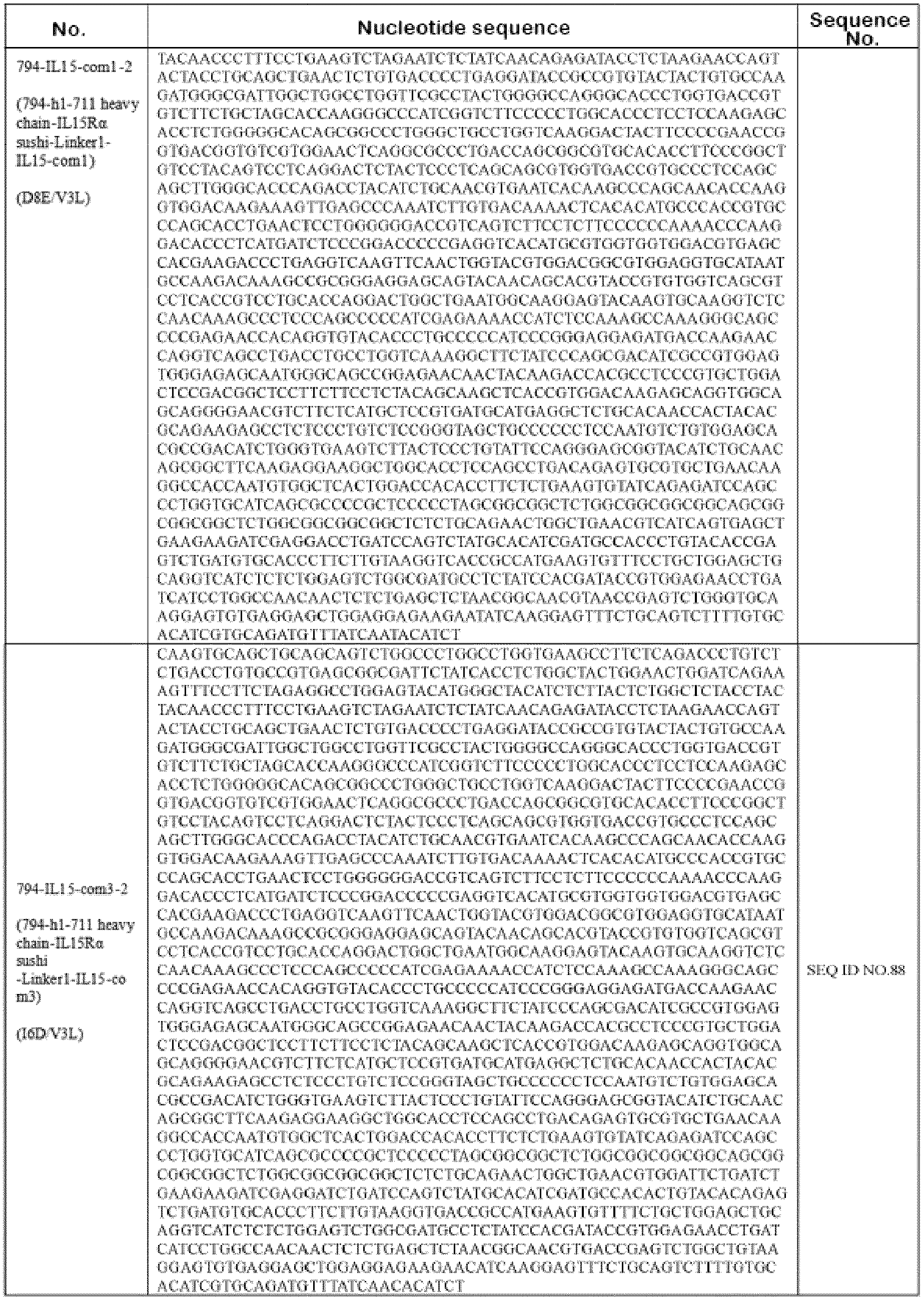

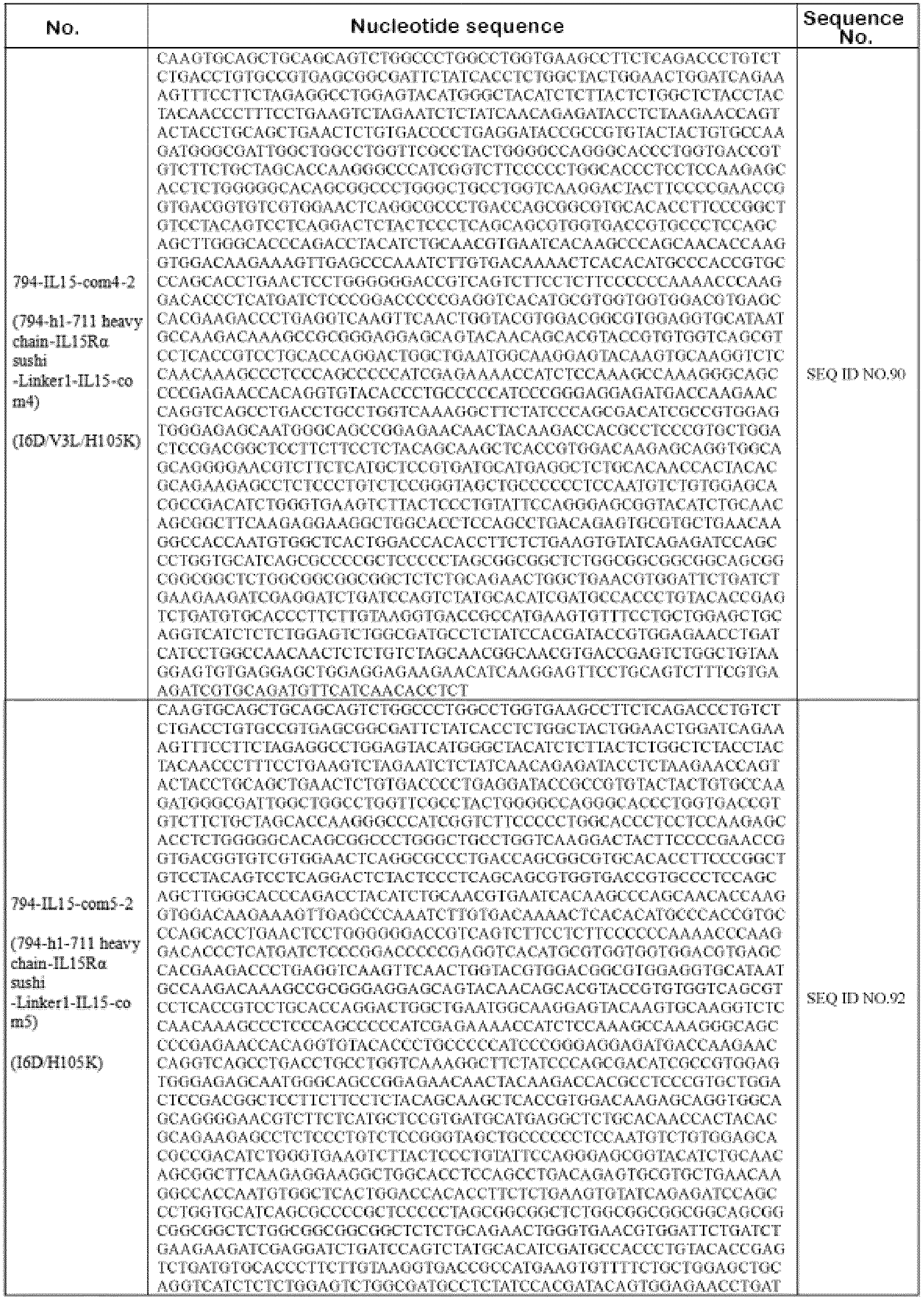

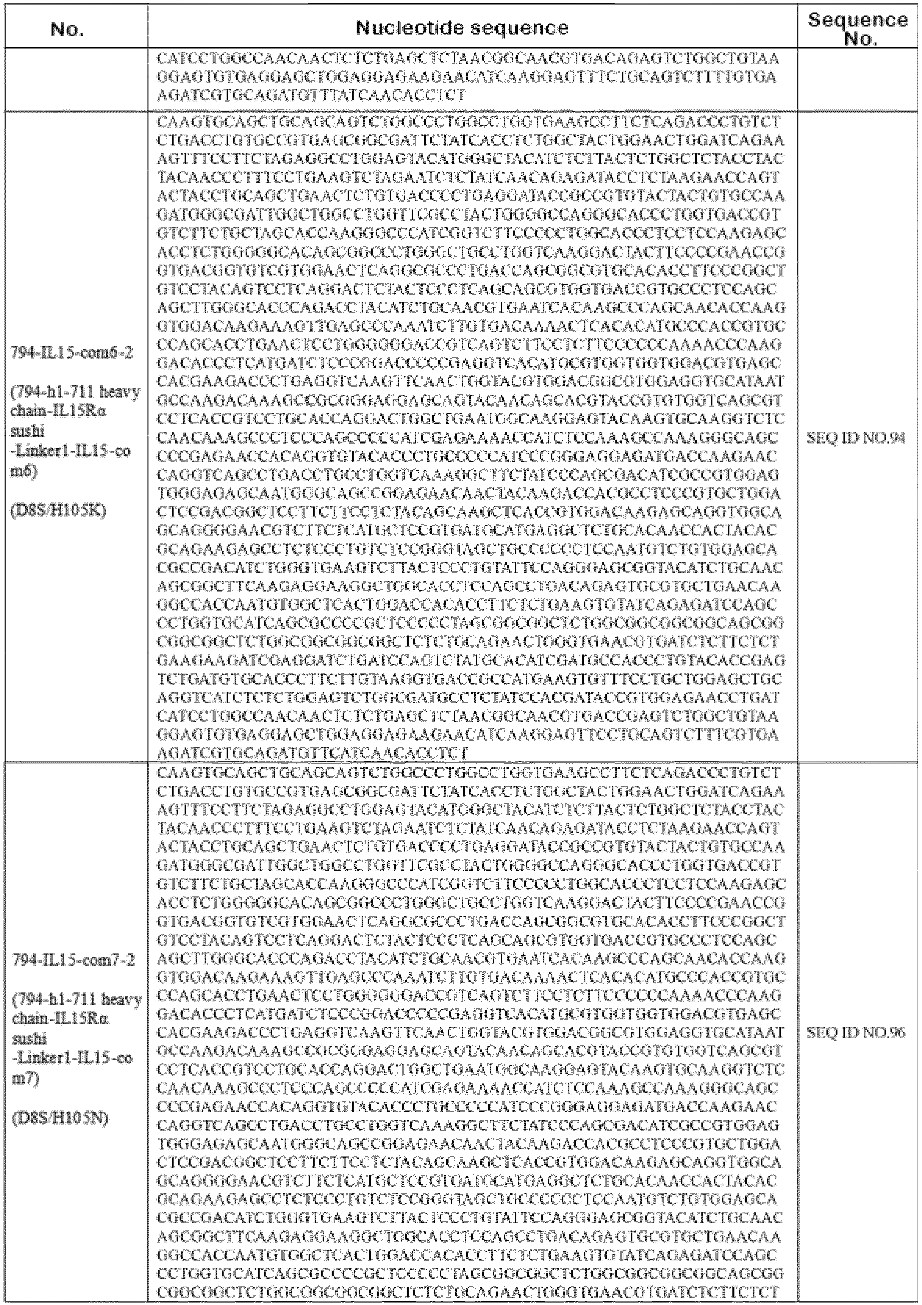

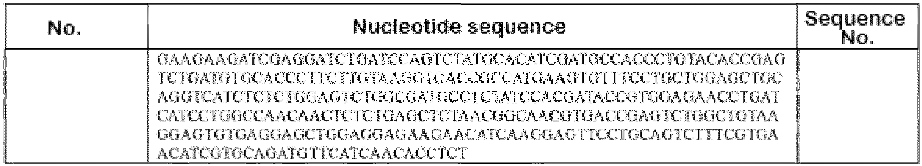

**Table 5. Molecular composition of PD-L1-IL-15 fusion protein**

| **Domains and corresponding amino acid sequences of PD-L1-IL-15 fusion protein in Examples 9-15** | | | | | |
|---|---|---|---|---|---|
| **Fusion protein No.** | **Sequence of monoclonal antibody heavy chain** | **IL15Rα sushi** | **Linker** | **IL-15 protein** | **Sequence of monoclonal antibody light chain** |
| T-IL15-WT-1 | SEQ ID NO.57 | SEQ ID NO.49 | NA | SEQ ID NO.1 | SEQ ID NO.59 |
| T-IL15-7-1 | SEQ ID NO.57 | SEQ ID NO.49 | NA | SEQ ID NO.3 | SEQ ID NO.59 |
| T-IL15-8-1 | SEQ ID NO.57 | SEQ ID NO.49 | NA | SEQ ID NO.5 | SEQ ID NO.59 |
| T-IL15-9-1 | SEQ ID NO.57 | SEQ ID NO.49 | NA | SEQ ID NO.7 | SEQ ID NO.59 |
| T-IL15-10-1 | SEQ ID NO.57 | SEQ ID NO.49 | NA | SEQ ID NO.9 | SEQ ID NO.59 |
| T-IL15-11-1 | SEQ ID NO.57 | SEQ ID NO.49 | NA | SEQ ID NO.1 1 | SEQ ID NO.59 |
| T-IL15-26-1 | SEQ ID NO.57 | SEQ ID NO.49 | NA | SEQ ID NO.13 | SEQ ID NO.59 |
| T-IL15-29-1 | SEQ ID NO.57 | SEQ ID NO.49 | NA | SEQ ID NO.15 | SEQ ID NO.59 |
| T-IL15-42-1 | SEQ ID NO.57 | SEQ ID NO.49 | NA | SEQ ID NO.17 | SEQ ID NO.59 |
| T-IL15-43-1 | SEQ ID NO.57 | SEQ ID NO.49 | NA | SEQ ID NO.19 | SEQ ID NO.59 |
| T-IL15-com1-1 | SEQ ID NO.57 | SEQ ID NO.49 | NA | SEQ ID NO.35 | SEQ ID NO.59 |
| T-IL15-com2-1 | SEQ ID NO.57 | SEQ ID NO.49 | NA | SEQ ID NO.37 | SEQ ID NO.59 |
| 794-IL15-WT-2 | SEQ ID NO.61 | SEQ ID NO.49 | SEQ ID NO.65 | SEQ ID NO.1 | SEQ ID NO.63 |
| 794-IL15-7-2 | SEQ ID NO.61 | SEQ ID NO.49 | SEQ ID NO.65 | SEQ ID NO.3 | SEQ ID NO.63 |
| 794-IL15-64-2 | SEQ ID NO.61 | SEQ ID NO.49 | SEQ ID NO.65 | SEQ ID NO.27 | SEQ ID NO.63 |
| 794-IL15-65-2 | SEQ ID NO.61 | SEQ ID NO.49 | SEQ ID NO.65 | SEQ ID NO.29 | SEQ ID NO.63 |
| 794-IL15-com1-2 | SEQ ID NO.61 | SEQ ID NO.49 | SEQ ID NO.65 | SEQ ID NO.35 | SEQ ID NO.63 |
| 794-IL15-com3-2 | SEQ ID NO.61 | SEQ ID NO.49 | SEQ ID NO.65 | SEQ ID NO.39 | SEQ ID NO.63 |
| 794-IL15-com4-2 | SEQ ID NO.61 | SEQ ID NO.49 | SEQ ID NO.65 | SEQ ID N0.41 | SEQ ID NO.63 |
| 794-IL15-com5-2 | SEQ ID NO.61 | SEQ ID NO.49 | SEQ ID NO.65 | SEQ ID NO.43 | SEQ ID NO.63 |
| 794-IL15-com6-2 | SEQ ID NO.61 | SEQ ID NO.49 | SEQ ID NO.65 | SEQ ID NO.45 | SEQ ID NO.63 |
| 794-IL15-com7-2 | SEQ ID NO.61 | SEQ ID NO.49 | SEQ ID NO.65 | SEQ ID NO.47 | SEQ ID NO.63 |

| | | | | | |
|---|---|---|---|---|---|
| Notes: The naming rules for IL-15 mutant fusion protein are as follows: for example: "T-IL15-xx-1": "T", indicating Tecentriq; "IL15-xx", indicating IL15-WT or IL15 mutant; "1", indicating the structural mode shown in Figure 5A; for example: "794-IL15-xx-2": "794", indicating 794-h1-71 mAb; "IL15-xx", indicating IL15-WT or IL15 mutant; "2", indicating the structural mode shown in Figure 5B. | | | | | |

**Table 6. Molecular composition of IL-15-Fc fusion protein**

| **Domains and corresponding amino acid sequences of IL-15-Fc fusion protein in Examples 9-16** | | | | | |
|---|---|---|---|---|---|
| **Fusion protein No.** | **IL-15 protein** | **Linker** | **IL15Rα sushi** | **Linker** | **Human Fc** |
| V9-IL15-61 (D8G) | SEQ ID NO.21 | SEQ ID NO.65 | SEQ ID NO.53 | SEQ ID NO.67 | SEQ ID NO.73 |
| V9-IL15-com6 (D8S/H105K) | SEQ ID NO.45 | SEQ ID NO.65 | SEQ ID NO.53 | SEQ ID NO.67 | SEQ ID NO.73 |
| V9-IL15-62 (D8I) | SEQ ID NO.23 | SEQ ID NO.65 | SEQ ID NO.53 | SEQ ID NO.67 | SEQ ID NO.73 |
| V9-IL15-63 (D8L) | SEQ ID NO.25 | SEQ ID NO.65 | SEQ ID NO.53 | SEQ ID NO.67 | SEQ ID NO.73 |
| V9-IL15-64 (I6P) | SEQ ID NO.27 | SEQ ID NO.65 | SEQ ID NO.53 | SEQ ID NO.67 | SEQ ID NO.73 |
| V9-IL15-65 (D8T) | SEQ ID NO.29 | SEQ ID NO.65 | SEQ ID NO.53 | SEQ ID NO.67 | SEQ ID NO.73 |
| V5-IL15-WT | SEQ ID NO.1 | SEQ ID NO.71 | SEQ ID NO.53 | SEQ ID NO.69 | SEQ ID NO.73 |
| V5-IL15-64 (I6P) | SEQ ID NO.27 | SEQ ID NO.71 | SEQ ID NO.53 | SEQ ID NO.69 | SEQ ID NO.73 |
| V5-IL15-65 (D8T) | SEQ ID NO.29 | SEQ ID NO.71 | SEQ ID NO.53 | SEQ ID NO.69 | SEQ ID NO.73 |

### Example 9. Expression of IL-15 Fusion Protein

Transient protein expression was performed by using the ExpiCHO expression system. Host cells, ExpiCHO-S (Cat no. A29127), passaged in ExpiCHOTM Expression Medium (Cat no. A2910001) were diluted to an appropriate density and placed on a shaker (100 rpm, 37°C, 8% CO₂) ready for transfection. Vectors carrying nucleic acid sequences encoding fusion proteins were added into OptiPRO^{™} SFM (Cat no.12309019) medium to achieve a final vector concentration of 0.5-1.0 µg/mL. An appropriate amount of ExpiFectamine^{™} CHO Reagent (Cat no.A29129) was added to OptiPRO^{™} SFM medium containing DNA to form DNA-ExpiFectamine^{™} CHO Reagent complexes, which were slowly instilled into the cell suspension to be transfected after being allowed to stand at room temperature for 1~5 min. On day 1 post-transfection, a certain amount of ExpiFectamine ^{™} CHO Enhancer (Cat no. A29129) and ExpiCHO ^{™} Feed (Cat no. A29129) were supplemented into the suspension, which was then cooled down to 32°C to continue the cultivation. On the 4th to 6th day post-transfection, a certain amount of ExpiCHO^{™} Feed (Cat no.A29129) was further added. On the 10th to 12th day post-transfection, the supernatant was harvested by centrifugation at 5000 g for 30 min.

### Example 10. Purification of IL-15 Fusion Protein

The mutant fusion protein was purified by magnetic beads. An appropriate amount of magnetic bead suspension (GenScript, Cat.NO.L00695) was added into the fermented supernatant and incubated in a rotary mixer for 2h to ensure the IL-15 fusion protein bound to the magnetic beads. After discarding the supernatant, the beads were washed three times with PBS. The IL-15 fusion protein was eluted with citrate at pH 3.0 and neutralized with 1M Tris-HCl. The concentration of IL-15 fusion protein was determined by NanoDrop One.

### Example 11. Purity of IL-15 fusion protein determined by size exclusion chromatography

The purity of the IL-15 mutant fusion protein of the present disclosure was determined by size exclusion chromatography (SEC) using a TSKgel G3000SWXI, column (TOSOH, 0008541) and a pre-column Tskgel guard columnSWXL (TOSOH, 0008543). The mobile phase (50mM PB, 300mM NaCl, pH6.8) was used to equilibrate the chromatographic column at a flow rate of 1mL/min. Ultraviolet detection wavelength was 280nm. The results are shown in table 7.

**Table 7. Purity of IL-15 fusion protein determined by size exclusion chromatography**

| **No.** | **SEC main peak purity %** | **No.** | **SEC main peak purity %** |
|---|---|---|---|
| T-IL15-7-1 | 96.99 | 794-IL15-com1-2 | 99.83 |
| T-IL15-8-1 | 93.7 | 794-IL15-com3-2 | 99.6 |
| T-IL15-9-1 | 93.4 | 794-IL15-com4-2 | 98.6 |
| T-IL15-10-1 | 96.5 | 794-IL15-com5-2 | 99.3 |
| T-IL15-11-1 | 94 | 794-IL15-com6-2 | 99.2 |
| T-IL15-26-1 | 99 | 794-IL15-com7-2 | 99.2 |
| T-IL15-29-1 | 95.6 | V9-IL15-61 | 97.7 |
| T-IL15-42-1 | 98.5 | V9-IL15-com6 | 97.5 |
| T-IL15-43-1 | 96.7 | V9-IL15-62 | 97.7 |
| T-IL15-WT-1 | 96.08 | V9-IL15-63 | 99.11 |
| 794-IL15-WT-2 | 98.3 | 794-IL15-65-2 | 98.56 |
| 794-IL15-7-2 | 99.78 | 794-IL15-64-2 | 98.32 |
| T-IL15-com1-1 | 98 | V5-IL15-WT | 98.23 |
| T-IL 15-com2-1 | 97.26 | | |

### Example 12. Proliferation assay of Mo7e cells induced by IL-15 mutant fusion protein

Mo7e is a human megakaryocytic leukemia cell line (Cobioer, CBP60791) that can be used to study effect of IL-15 on cell proliferation activity. IL-15 mutant fusion protein was diluted with 1640-10% FBS (RPMI1640, Gibco, 72400047; FBS, Gibco, 10099141) to a gradient concentration (final concentration: 100000 pM-1.28 pM, 5-fold dilution). Mo7e cells were diluted with 1640-10% FBS to 10⁵ cells/ml. 50 µl mutant culture medium and 50 µl Mo7e cell suspension were added to a 96-well U-bottom plate, and then mixed and incubated at 37°C with 5% CO₂. 72 hours later, the culture plate was taken out and added with 100 µl Cell Titer Glo luminescent cell viability detection reagent (Promega, G7571) to detect the fluorescence intensity, which was proportional to the cell proliferation ability.

The results are shown in Figures **6A-6D****:** T-IL15-7-1, T-IL15-8-1 and T-IL15-9-1 mutants show significantly reduced activity compared to T-II,15-WT-1, with an EC50 of 5735 pM for the T-IL15-7-1 and 133.3 pM for the corresponding T-II,15-WT-1 (Figure 6A). T-IL15-10-1, T-IL15-11-1 and T-IL15-26-1 mutants show significantly reduced activity compared to T-II,15-WT-1, with an EC50 of 28076 pM for the T-IL15-10-1 and 290.9 pM for the T-IL15-26-1, respectively and an EC50 of 143.3 pM for the corresponding T-II,15-WT-1 (Figure 6B). T-IL15-29-1 and T-IL15-42-1 mutants show reduced activity compared to T-II,15-WT-1, with an EC50 of 285.2 pM and 194.5 pM, respectively, and T-IL15-43-1 mutant shows enhanced activity compared to T-II,15-WT-1, with an EC50 of 103.2 pM for the T-IL15-43-1 mutant and 150.7 pM for the T-IL15-WT-1 (Figure 6C). T-IL15-com1-1 and T-IL15-com2-1 combined mutant show reduced activity compared to T-IL15-WT-1; Tecentriq control has no effect on Mo7e cell proliferation, ***i.e.* anti-PD-L1 antibody does not have proliferation-inducing activity on Mo7e cells (****Figure 6 D)****, and the effect on cell proliferation activity is produced by IL-15.**

### Example 13. CD8⁺T cell proliferation induced by IL15 mutant fusion protein

In order to detect the effect of IL-15 mutant fusion protein on stimulating the proliferation of CD8+ T cells in human PBMCs (Allcells, Lot: 1911150123), Ki67 was used as a proliferation marker in this example to detect the proliferation proportion of CD8+ T cells after stimulating human PBMCs with IL-15 mutant fusion protein at different concentrations for three days. First, human PBMCs were suspended in RPMI1640 medium (Gibco, Cat: 72400047) containing 10% FBS (Gibco, Cat: 10099141) and 1% penicillin-streptomycin (Gibco, Cat: 15140122). After adjusting the cell density to 2 × 10⁶ cells/ml, the cell suspension was added to a 96-well U-bottom plate (Corning, Cat: 3799) at 100 µl/well. The IL-15 mutant fusion protein was then diluted at a 4-fold gradient from 500 nM, with a total of 11 gradients, after which 100 µl/well of samples of each concentration were added to a 96-well U-bottom plate coated with human PBMCs, mixed evenly, and cultured in an incubator with 5% CO₂ at 37°C for three days. The cultured cells were stained with LIVE/DEAD Fixable Violet Dead Cell Stain Kit (Invitrogen, Cat: L34964) and CD3-AF700 (BD, Cat: 557943), CD8-FITC (BD, Cat: 555366), and APC-Ki67 (Biolegend, Cat: 350514) antibodies, followed by using flow cytometry to detect the proportion of Ki67+ cells in CD3+ CD8+ T cells stimulated with IL-15 mutant fusion protein at different concentrations. **Figures 7A-7N** show the proliferation proportion of CD8+ T cells after human PBMCs were stimulated with gradiently diluted T-IL15-7-1, T-IL15-8-1, T-IL15-9-1, T-IL15-10-1, T-IL15-11-1, T-IL15-26-1, T-IL15-29-1, T-IL15-42-1, T-IL15-43-1, 794-IL15-7-2, 794-IL15-com1-2, 794-IL15-com3-2, 794-IL15-com4-2, 794-IL15-com5-2, 794-IL15-com6-2, 794-IL15-com7-2, V9-IL15-61, V9-IL15-62, V9-IL15-63, V9-IL15-com6, 794-IL15-65-2, 794-IL15-64-2, V5-IL15-WT and P22339 for three days, as well as EC50 values of proliferation stimulated by each mutant. Among them, T-IL15-8-1, T-IL15-9-1 and T-IL15-11-1 show a significantly reduced effect on proliferation ability of CD8+ T cells which does not reach the platform so that the curve could not be fitted to calculate the EC50; 794-IL15-com4-2, 794-IL15-com5-2, 794-IL15-com6-2, 794-IL15-com7-2, V9-IL15-61, V9-IL15-62, V9-IL15-63, V9-IL15-com6 and 794-IL15-65-2 do not reach the platform so that the EC 50 values are not accurately fitted; however, the results show that the combination of these mutants exhibit a reduced effect on proliferative activity of CD8+ T cells compared to 794-IL15-WT-2 or P22339. T-IL15-WT-1 and 794-IL15-WT-2 are wild-type IL-15 controls, and P22339 is mutant IL-15 control; 794-h1-71 monoclonal antibody is anti-PD-L1 antibody control; DrugO is negative control without adding any protein. Compared with wild-type IL-15 controls, IL15 mutants show a weaker effect on proliferation of CD8+ T cell. The 794-h1-71 mAb control group has no effect on CD8+ T cell proliferation, *i.e.* **anti-PD-L1 antibody does not have induce proliferation activity on CD8+ T cells (****Figure 7B****) and the effect on cell proliferative activity is produced by IL-15.**

### Example 14. NK cell proliferation induced by IL15 mutant fusion protein

In order to detect the effect of IL-15 mutant fusion protein on stimulating the proliferation of NK cells in human PBMCs (Allcells, Lot: 1911150123), Ki67 was used as a proliferation marker in this example to detect the proliferation proportion of NK cells after stimulating human PBMCs with IL-15 mutant fusion protein at different concentrations for three days. Firstly, human PBMCs were suspended in RPMI1640 medium (Gibco, Cat: 72400047) containing 10% FBS (Gibco, Cat: 10099141) and 1% penicillin-streptomycin (Gibco, Cat: 15140122). After adjusting the cell density to 2 × 10⁶ cells/ml, the cell suspension was added to a 96-well U-bottom plate (Corning, Cat: 3799) at 100 µl/well. The IL-15 mutant fusion protein was then diluted at a 4-fold gradient from 500nM, with a total of 11 gradients, after which 100 µl/well of samples of each concentration were added to a 96-well U-bottom plate coated with human PBMCs had, mixed evenly, and cultured in an incubator with 5% CO₂ at 37°C for three days. The cultured cells were stained with LIVE/DEAD Fixable Violet Dead Cell Stain Kit (Invitrogen, Cat: L34964) and CD3-AF700 (BD, Cat: 557943), CD56-PE (BD, Cat: 555516), and APC-Ki67 (Biolegend, Cat: 350514) antibodies, followed by using Invitrogen Attune NxT flow cytometer to detect the proportion of Ki67+ cells in NK cells stimulated with IL-15 mutant fusion protein at different concentrations. Figures 8A-8M show the proliferation proportion of NK cells (CD3⁻CD56⁺) after human PBMCs were stimulated with gradiently diluted T-IL15-7-1, T-IL15-8-1, T-IL15-9-1, T-IL15-10-1, T-IL15-11-1, T-IL15-26-1, T-IL15-29-1, T-IL15-42-1, T-IL15-43-1, 794-IL15-7-2, 794-IL15-com1-2, 794-IL15-com4-2, 794-IL15-com5-2, 794-IL15-com6-2, 794- IL15 -com7-2, V9-IL15-61, V9-IL15-62, V9-IL15-63, V9-IL15-com6, 794-IL15-65-2, 794-IL15-64-2, V5-IL15-WT and P22339 for three days, as well as EC50 values of proliferation stimulated by each mutant. Among them, T-IL15-8-1, T-IL15-9-1, T-IL15-11-1, V9-IL15-com6, V9-IL15-62 and V9-IL15-63 show a significantly reduced effect on proliferation ability of NK cells which does not reach the platform so that the EC 50 values are not accurately fitted; however, the combination of these mutants show a significantly reduced effect on proliferative activity of NK cells compared to T-II,15-WT-1 or P22339 control groups. T-IL15-WT-1 and 794-IL15-WT-2 are wild-type IL-15 controls, and P22339 is mutant IL-15 control; 794-h1-71 monoclonal antibody is anti-PD-L1 antibody control; DrugO is negative control without adding any protein. Compared with wild-type IL-15 controls, IL-15 mutants show a weaker effect on proliferation of NK cells. The 794-h1-71 mAb control group has no effect on NK cell proliferation, *i.e.* **anti-PD-L1 antibody does not induce proliferation activity on NK cells (****Figure 8B****) and the effect on cell proliferative activity is produced by IL-15.**

### Example 15. In vivo pharmaceutical efficacy of humanized anti-PD-L1 antibody-Il15 bifunctional Mmolecule/fusion protein in mice

Human melanoma A375 cells (Beina Bio; BNCC100266), 5×10⁶ cells/100 µL, were inoculated subcutaneously in the right back of NPG mice. On the next day after inoculation with A375, cryopreserved PBMCs (ALLCELLs, Fig. PB005F-C) were resuscitated and injected into the NPG mice (5-6 weeks old, female; purchased from Beijing Vitalstar Biotechnology Co.,Ltd.) via tail vein at a dose of 5 × 10⁶ cells/200 µl. Six days after PBMC inoculation, 40 µl blood was collected to detect the proportion of hCD45+ cells. After the tumors grew to about 80 mm³, mice with body weight, proportion of hCD45+ cells and tumor volume that were too large and too small were excluded. According to the tumor volume, the mice were randomly divided into 4 groups, including PBS group, Tecentriq group (10 mg/kg), 794-IL15-WT-2 group (1 mg/kg) and 794-IL15-com6-2 group (4 mg/kg), with 8 mice in each group and 32 mice in total. Drugs were administered intraperitoneally once weekly for a total of 1 dose in 794-IL15-WT-2 group (1 mg/kg) and 794-IL15-com6-2 group (4 mg/kg), and twice weekly for a total of 5 doses in PBS group and Tecentriq group (10 mg/kg). Tumor volumes were measured 3 times a week, and the data were recorded. Tumor volume (long diameter × short diameter²/2) and tumor growth inhibition rate (TGI_{TV} (%), TGI_{TV} (%) = [1- (Ti-T0)/(Vi-V0)]× 100%; Ti: mean tumor volume of the treatment group on day i of administration, T0: mean tumor volume of the treatment group on day 0 of administration; Vi: mean tumor volume of vehicle control group on day i of administration; V0: mean tumor volume of vehicle control group on day 0 of administration) were calculated. On day 14 of group administration, the drug administration groups all had significant inhibitory effect on tumor volume, with a statistical difference (P < 0.05), and the 794-IL15-WT-2 group and 794-IL15-com6-2 group had significant inhibitory effect on tumor volume compared to Tecentriq group (P < 0.05). See Figures **9A-9C** and Table 8.

**Table 8. Effect of tested drugs on A375 tumor volume in immune-reconstituted NPG mice**

| **Group** | **Tested drug** | **Tumor volume (mm³)^{a}** | | | **P^{b}** | **P^{c}** |
|---|---|---|---|---|---|---|
| | | **Before administr ation** | **On day 14 of group administrat ion** | **TGI_{TV} (%)** | | |
| G1 | PBS | 82±6 | 427±73 | - | - | - |
| G2 | Tecentriq | 78±5 | 253±38 | 49.35 | <0.0001 | - |
| G3 | 794-IL15-WT-2 | 78±4 | 198±35 | 65.08 | <0.0001 | 0.0399 |
| G4 | 794-IL15-com6-2 | 78±5 | 129±34 | 85.15 | <0.0001 | 0.0125 |

| | | | | | | |
|---|---|---|---|---|---|---|
| Notes: a: mean ± standard error; b: statistical comparison of tumor volume between drug administration group and vehicle control group (PBS) on day 14 of group administration, Two-way ANOVA analysis, P < 0.05, P < 0.01, P < 0.001, P < 0.0001; c: statistical comparison of tumor volume between drug administration group and Tecentriq (positive drug) group on day 14 of group administration, Two-way ANOVA analysis, P < 0.05, P < 0.01, P < 0.001, P < 0.0001. | | | | | | |

The above results indicate that both 794-IL15-WT-2 and 794-IL15-com6-2 (the humanized anti-PD-L1 antibody-IL15 bifunctional molecule/fusion protein) have a significant inhibitory effect on the growth of subcutaneously xenografted A375 tumor (P < 0.0001); 794-IL15-WT-2 and 794-IL15-com6-2 groups have a stronger inhibitory effect on tumor volume compared to the Tecentriq group, with a significant difference (P < 0.05).

### Example 16. In vivo pharmaceutical efficacy of IL15-Fc fusion protein in mice

Human melanoma A375 cells (Beina Bio; BNCC100266), 5×10⁶ cells/100 µL, were inoculated subcutaneously in the right back of NPG mice. On the next day after inoculation with A375, cryopreserved PBMCs (ALLCELLs, Fig. PB005F-C) were resuscitated and injected into NPG mice (5-6 weeks old, female; purchased from Beijing Vitalstar Biotechnology Co.,Ltd.) via tail vein at a dose of 5 × 10⁶ cells/200 µl. Six days after PBMC inoculation, 40 µl blood was collected to detect the proportion of hCD45+ cells. After the tumors grew to about 69 mm³, mice with body weight, proportion of hCD45+ cells and tumor volume that were too large and too small were excluded. According to the tumor volume, the mice were randomly divided into 7 groups, including PBS, ALT803 (0.2 mg/kg), V9-IL15-61 (1 mg/kg), V9-IL15-61 (5 mg/kg), V9-IL15-com6 (1 mg/kg), V9-IL15-com6 (5 mg/kg) and V5-IL15-WT (2 mg/kg) groups, with 8 mice in each group and 56 mice in total. Drugs were administered intraperitoneally once weekly for a total of 3 doses according to groups. Tumor volumes were measured 3 times a week, and the data were recorded. Tumor volume (long diameter × short diameter²/2) and tumor growth inhibition rate (TGI_{TV} (%), TGI_{TV} (%) = [1- (Ti-T0)/(Vi-V0)]× 100%; Ti: mean tumor volume of the treatment group on day i of administration, T0: mean tumor volume of the treatment group on day 0 of administration; Vi: mean tumor volume of vehicle control group on day i of administration; V0: mean tumor volume of vehicle control group on day 0 of administration) were calculated. On day 21 of group administration, compared with PBS control group, the drug administration groups all had significant inhibitory effect on tumor volume, with a statistically significant difference (P < 0.05), and the ALT803 (positive drug) group and V9-IL15-com6 group had similar inhibitory effects on tumor volume (P > 0.05). See **Figures 10A-10C** and Table 9.

**Table 9. Effect of tested drugs on A375 tumor volume in immune-reconstituted NPG mice**

| **Group** | **Tested drug** | **Tumor volume (mm³)^{a}** | | | **P^{b}** |
|---|---|---|---|---|---|
| | | **Before administration** | **Day 21 of group administration** | **TGI_{TV} (%)** | |
| G1 | PBS | 70 ±5 | 1755±360 | - | - |
| G2 | ALT803 (0.2mg/kg) | 68 ±5 | 788 ±144 | 57.3 | <0.0001 |
| G3 | V9-IL15-61 (1 mg/kg) | 69 ±6 | 1082 ±137 | 39.9 | <0.0001 |
| G4 | V9-IL15-61 (5 mg/kg) | 70 ±6 | 1001 ±206 | 44.7 | <0.0001 |
| G5 | V9-IL15-com6 (1 mg/kg) | 69 ±6 | 1144 ±298 | 36.2 | <0.0001 |
| G6 | V9-IL15-com6 (5 mg/kg) | 67 ±6 | 809 ±215 | 56.0 | <0.0001 |
| G7 | V5-IL15-WT (2 mg/kg) | 69 ±6 | 1065 ±148 | 40.9 | <0.0001 |

| | | | | | |
|---|---|---|---|---|---|
| Notes: a: mean ± standard error; b: statistical comparison of tumor volume between drug administration group and vehicle control group (PBS) on day 14 of group administration, Two-way ANOVA analysis, P < 0.05, P < 0.01, P < 0.001, P < 0.0001; c: the tumor volume on day 21 is analyzed by identify outliers, and one data in V9-IL15- com6 5mpk group was unusually large and was excluded. | | | | | |

The experimental animals are in a good state of activity and eating during administration. After administration, the mice in ALT803 and V5-IL15-WT groups show a significant decrease in body weight, and some mice are even dead, which indicates that mice are intolerant to ALT803 and V5-IL15-WT at this dose and frequency. Animal death occurs in the V9-II,15-61 (5mpk) group and V9-IL15-com6 (1mpk) group, but GVHD condition such as anemia is observed before death. It is speculated that the death of the mice is caused by GVHD and irrelevant to the drugs. See Figure 10D, Table 10 and Table 11.

**Table 10. Effects of tested drugs on body weight of immune-constituted A375 tumor-bearing NPG mice**

| **Group** | **Tested drug** | **Body weight (g)^{a}** | | | **Body weight change on day 21 of administration (g)** |
|---|---|---|---|---|---|
| | | **Before administration** | **On day 21 of group administratio n** | **P^{b}** | |
| G1 | PBS | 21.7±0.7 | 21.3±0.8 | - | -0.4 |
| G2 | ALT803 (0.2 mg/kg) | 21.7±0.5 | 20.8±1.0 | 0.6482 | -0.9 |
| G3 | V9-IL15-61 (1 mg/kg) | 20.8±0.5 | 20.8±0.7 | 0.3405 | 0 |
| G4 | V9-IL15-61 (5 mg/kg) | 21.4±0.7 | 19.6±0.9 | 0.2574 | -1.8 |
| G5 | V9-IL15-com6 (1 mg/kg) | 21.7±0.7 | 20.8±1.0 | 0.4836 | -0.9 |
| G6 | V9-IL15-com6 (5 mg/kg) | 21.3±0.8 | 18.4±1.1 | 0.0097 | -2.9 |
| G7 | V5-IL15-WT (2 mg/kg) | 21.5±0.3 | 20.0±0.7 | 0.0037 | -1.5 |

| | | | | | |
|---|---|---|---|---|---|
| Notes: a: mean ± standard error; b: statistical comparison of body weight between drug administration group and vehicle control group on day 21 of group administration, Two-way ANOVA analysis. | | | | | |

**Table 11. Effects of tested drugs on survival of immune-constituted A375 tumor-bearing NPG mice**

| **Group** | **Tested drug** | **Number of surviving mice on day 21 of group administration ^{a}** | **Death time of mouse** | | | |
|---|---|---|---|---|---|---|
| | | | **Day 15 of group administration** | **Day 19 of group administration** | **Day 20 of group administration** | **Day 21 of group administration** |
| G1 | PBS | 8 | - | - | - | - |
| G2 | ALT803 (0.2mg/kg) | 7 | 1^{b} | - | - | - |
| G3 | V9-IL15-61 (1 mg/kg) | 8 | - | - | - | - |
| G4 | V9-IL15-61 (5 mg/kg) | 7 | 0 | - | - | - |
| G5 | V9-IL15-com6 (1 mg/kg) | 7 | - | - | 0 | - |
| G6 | V9-IL15-com6 (5 mg/kg) | 8 | - | - | - | - |
| G7 | V5-IL15-WT (2 mg/kg) | 6 | - | 1 | - | 0 |

| | | | | | | |
|---|---|---|---|---|---|---|
| Notes: a: mean ± standard error; b: mice died after group administration, 1 represents that 1 mouse died on the day, 0 represents that 1 mouse died on the day but anemia, jaundice and other symptoms were observed before death. | | | | | | |

The above results show that IL15-Fc fusion proteins, both V9-II,15-61 and V9-IL15-com6, have a significant inhibitory effect on the growth of subcutaneously xenografted, human immune-reconstituted A375 tumor. V9-IL15-com6 (5 mg/kg) shows a comparable level of TGI (tumor growth inhibition rate) but with better safety compared to the positive control antibody ALT803 (0.2mg/kg).

## Claims

1. An IL-15 mutant polypeptide, comprising mutation(s) at one or more amino acid residues corresponding to Val3, Ile6, Asp8, or His105 of wild-type IL-15.

2. A polypeptide comprising an IL-15 mutant, wherein the polypeptide comprises mutation(s) at one or more amino acid residues corresponding to Val3, Ile6, Asp8, or His105 of wild-type IL-15.

3. The polypeptide according to claim 1 or 2, wherein the mutation(s) is substitution, insertion or deletion;
preferably, the polypeptide comprises at least mutations at two, three or four amino acid residues at Val3, Ile6, Asp8, or His105;
preferably, the mutation is selected from amino acid substitution of the group of: Val3Leu (V3L), Ile6Asp (I6D), Ile6Pro (I6P), Asp8Glu (D8E), Asp8Gln (D8Q), Asp8Arg (D8R), Asp8Ser (D8S), Asp8Val (D8V), Asp8Gly (D8G), Asp8Ile (D8I), Asp8Leu (D8L), Asp8Thr (D8T), His105Asn (H105N), and/or His105Lys (H105K);
preferably, the polypeptide comprises mutation or combination of mutations of:
(1) Asp8Glu, (2) Asp8Gln, (3) Asp8Arg, (4) Asp8Ser, (5) Asp8Val, (6) Val3Leu, (7) Ile6Asp, (8) His105 Lys, (9) His105 Asn, (10) Asp8Gly, (11) Asp8Ile, (12) Asp8Leu, (13) Ile6Pro, (14) Asp8Thr, (15) Asp8Glu and Val3Leu, (16) Asp8Glu and Ile6Asp, (17) Val3Leu and Ile6Asp, (18) Ile6Asp and His105Lys, (19) Asp8Ser and His105Lys, (20) Asp8Ser and His105Asn, or (21) Val3Leu, Ile6Asp and His105Lys;
preferably, the IL-15 mutant has at least 80%, 85%, 90%, 91%, 92%, 93%, 94%, 95%, 96%, 97%, 98%, or 99% sequence identity to human wild-type IL-15.

4. The polypeptide according to any one of claims 1-3, wherein the amino acid sequence of the IL-15 mutant is as shown in SEQ ID NO.3, SEQ ID NO.5, SEQ ID NO.7, SEQ ID NO. NO.9, SEQ ID NO. 11, SEQ ID NO. 13, SEQ ID NO.15, SEQ ID NO.17, SEQ ID NO. 19, SEQ ID NO.21, SEQ ID NO.23, SEQ ID NO. 25. SEQ ID NO.27, SEQ ID NO.29, SEQ ID NO.35, SEQ ID NO.37, SEQ ID NO.39, SEQ ID NO.41, SEQ ID NO.43, SEQ ID NO.45 or SEQ ID NO.47;
preferably, the polypeptide has activities of:
(1) mediating proliferation of human CD8+ T cells;
(2) mediating proliferation of human NK cells; and/or,
(3) inhibiting tumor growth;
preferably, the polypeptide has activity of mediating proliferation/expansion of CD8+ T cells and/or NK cells which is lower than that of a polypeptide comprising a wild-type IL-15;
preferably, the wild-type IL-15 has an amino acid sequence as shown in SEQ ID NO. 1.

5. A protein comprising the polypeptide of any one of claims 1-4, wherein the protein further comprises: (1) an immunoglobulin molecule or part thereof fused to the IL-15 mutant, and/or (2) IL-15Rα fused to the IL-15 mutant;
preferably, the immunoglobulin molecule is an antibody or an antigen-binding fragment; the part of the immunoglobulin molecule is an immunoglobulin Fc region;
preferably, the antibody or antigen-binding fragment is selected from:
(1) a chimeric antibody or fragment thereof;
(2) a humanized antibody or fragment thereof; or,
(3) a fully humanized antibody or fragment thereof;
preferably, the antibody or antigen-binding fragment is selected from one or more of F(ab)₂, Fab', Fab, Fv, scFv, bispecific antibody, nanobody and antibody minimum recognition unit;
preferably, the immunoglobulin Fc region is selected from a Fc region of any one of IgG1, IgG2, IgG3, IgG4, IgA, IgM, IgE or IgD; preferably, a sequence comprising a constant region of human or murine IgG1, IgG2, IgG3 or IgG4 antibody; preferably, the immunoglobulin Fc region has an amino acid sequence as shown in SEQ ID NO.73; preferably, the IL-15 mutant is fused to the immunoglobulin molecule or part thereof with or without a linker peptide, or the IL-15 mutant is fused to the IL-15Rα with or without a linker peptide; preferably, a linker peptide is used; preferably, the linker peptide as shown in SEQ ID NO.65, SEQ ID NO.67, SEQ ID NO.69, or SEQ ID NO.71 is used;
preferably, the IL-15 mutant is fused to the IL-15Rα and then to the immunoglobulin molecule or part thereof with or without a linker peptide; preferably, a linker peptide is used; preferably, the linker peptide as shown in SEQ ID NO.65, SEQ ID NO.69, or SEQ ID NO.71 is used.

6. The protein according to claim 5, wherein individual domains are connected from N-terminus to C-terminus in order of:
(1) the immunoglobulin protein molecule or part thereof, the IL-15Rα, and the IL-15 mutant;
(2) the immunoglobulin molecule or part thereof, the IL-15 mutant, and the IL-15Rα;
(3) the IL-15 mutant, the IL-15Rα, and immunoglobulin molecule or part thereof;
(4) the IL-15Rα, IL-15 mutant, and the immunoglobulin molecule or part thereof;
(5) the IL-15 mutant, and the immunoglobulin molecule or part thereof;
(6) the immunoglobulin molecule or part thereof, and the IL-15 Rα;
(7) the IL-15Rα, and the immunoglobulin molecule or part thereof;
(8) the IL-15 mutant, and the IL-15Rα; or,
(9) the IL-15Rα, and the IL-15 mutant;
preferably, the IL-15Rα or IL-15 mutant is fused to N-terminus of a variable region of a heavy chain of the immunoglobulin molecule or C-terminus of the immunoglobulin Fc region when the IL-15Rα or IL-15 mutant is fused to the immunoglobulin molecule; and the IL-15Rα or IL-15 mutant is fused to N-terminus or C-terminus of the immunoglobulin Fc region when the IL-15Rα or IL-15 mutant is fused to the immunoglobulin Fc region.

7. A protein comprising:
(1) an immunoglobulin heavy chain;
(2) an immunoglobulin light chain;
(3) an IL-15Rα; and,
(4) the IL-15 mutant polypeptide of any one of claims 1-4;
preferably, the protein is a homodimer comprising a monomer composed of (1) - (4); preferably, the IL-15Rα is fused to N-terminus of a variable region of the immunoglobulin heavy chain or C-terminus of an immunoglobulin Fc region with or without a linker peptide;
preferably, the IL-15 mutant polypeptide is non-covalently linked to the IL-15Rα, or the IL-15 mutant is fused to the other end of the IL-15Rα with or without a linker peptide.

8. A protein comprising:
(1) an immunoglobulin Fc region;
(2) an IL-15Rα; and,
(3) the IL-15 mutant polypeptide of any one of claims 1-4;
preferably, the protein is a homodimer comprising monomers composed of (1) - (3); preferably, the IL-15Rα is fused to N-terminus or C-terminus of the IL-15 mutant polypeptide with or without a linker peptide, and then fused to N-terminus or C-terminus of the immunoglobulin Fc region with or without a linker peptide.

9. The protein according to any one of claims 5-8, wherein the immunoglobulin is selected from an anti-PD-L1 antibody; preferably, the anti-PD-L1 antibody is Tecentriq, KN-035, or 794-h1-71;
preferably, the anti-PD-L1 antibody comprises a heavy chain variable region and a light chain variable region, wherein the heavy chain variable region and the light chain variable region have sequences shown in SEQ ID NO: 99 and SEQ ID NO: 100, respectively, or sequences having at least 80%, 85%, 90%, 95%, 96%, 97%, 98%, 99%, or greater identity to the sequences shown in SEQ ID NO: 99 and SEQ ID NO: 100; or, the heavy chain variable region and light chain variable region have sequences shown in SEQ ID NO: 97 and SEQ ID NO: 98, respectively, or sequences having at least 80%, 85%, 90%, 95%, 96%, 97%, 98%, 99% or greater identity to the sequences shown in SEQ ID NO: 97 and SEQ ID NO: 98;
preferably, the IL-15Rα is selected from IL-15Rα-sushi; preferably, the IL-15Rα-sushi has an amino acid sequence as shown in SEQ ID NO.49, SEQ ID NO.51, SEQ ID NO.53, or SEQ ID NO.55.

10. An antibody or antigen-binding fragment capable of specifically binding to PD-L1, wherein the antibody or antigen-binding fragment comprises a heavy chain variable region and a light chain variable region; preferably, the heavy chain variable region and the light chain variable region have the sequences shown in SEQ ID NO: 99 and SEQ ID NO: 100, respectively, or sequences having at least 80%, 85%, 90%, 95%, 96%, 97%, 98%, 99% or greater identity to the sequences shown in SEQ ID NO: 99 and SEQ ID NO: 100;
preferably, the antibody or antigen binding fragment binds to human programmed death-ligand 1 (PD-L1) at a dissociation constant (KD) of 1.8 × 10⁻⁹ M or less, and the antibody or antigen binding fragment binds to cynomolgus monkey programmed death-ligand 1 (PD-L1) at a dissociation constant (KD) of 9.4 × 10⁻¹⁰ M or less; or optionally, the antibody or antigen-binding fragment binds to or does not bind to monkey PD-L1;
optionally, the antibody or antigen-binding fragment binds to or does not bind to murine PD-L1;
preferably, the anti-PD-L1 antibody competitively binds to PD-L1 or epitope thereof, and has activities of:
(1) specifically binding to a recombinant PD-L1 protein and a cell expressing PD-L1;
(2) blocking binding of PD-L1 to PD-1 protein;
(3) inhibiting binding of PD-1 to PD-L1 expressed on cell surface;
(4) enhancing T cell activity; or/and
(5) inhibiting tumor growth;
preferably, the anti-PD-L1 antibody comprises a constant region derived from any one of IgG1, IgG2, IgG3, IgG4, IgA, IgM, IgE or IgD; preferably, a sequence comprising a constant region of human or murine IgG1, IgG2, IgG3, or IgG4 antibody;
preferably, the PD-L1 antibody is selected from one or more of F(ab)₂, Fab', Fab, Fv, scFv, and bispecific antibody.

11. An isolated nucleic acid molecule encoding the polypeptide of any one of claims 1-4, the protein of any one of claims 5-9, or the antibody or antigen-binding fragment of claim 10.

12. An expression vector comprising the isolated nucleic acid molecule of claim 11.

13. A host cell comprising the isolated nucleic acid molecule of claim 11 or the expression vector of claim 12; preferably, the host cell is a eukaryotic cell or prokaryotic cell; more preferably, the host cell is derived from mammals, yeasts, insects, *Escherichia coli* and/or *Bacillus subtilis*; more preferably, the host cell is selected from Chinese hamster ovary (CHO) cells.

14. A method for preparing a polypeptide or protein, comprising culturing the host cell of claim 13 under an appropriate condition, and isolating the polypeptide or protein.

15. A pharmaceutical composition comprising the polypeptide of any one of claims 1-4, the protein of any one of claims 5-9, the antibody or antigen-binding fragment of claim 10, the isolated nucleic acid molecule of claim 11, the expression vector of claim 12, the host cell of claim 13, or the product prepared by the method of claim 14; and a pharmaceutically acceptable carrier; preferably, the pharmaceutical composition further comprises an additional anti-tumor agent.

16. Use of the polypeptide of any one of claims 1-4, the protein of any one of claims 5-9, the antibody or antigen-binding fragment of claim 10, the isolated nucleic acid molecule of claim 11, the expression vector of claim 12, the host cell of claim 13, the product prepared by the method of claim 14, or the pharmaceutical composition of claim 15 in the manufacture of a medicament for the prevention and/or treatment of a disease in an individual; preferably, the disease is a tumor or inflammatory disease;
preferably, the tumor is selected from glioblastoma, prostate cancer, blood cancer, B cell tumor, multiple myeloma, B cell lymphoma, B cell non-Hodgkin lymphoma, Hodgkin lymphoma, chronic lymphocytic leukemia, acute myeloid leukemia, cutaneous T cell lymphoma, T cell lymphoma, solid tumor, urothelial/bladder cancer, melanoma, lung cancer, renal cell carcinoma, breast cancer, gastric cancer and esophageal cancer, prostate cancer, pancreatic cancer, colorectal cancer, ovarian cancer, non-small cell lung cancer and squamous cell head and neck cancer.

17. A method for preventing and/or treating a disease in an individual, comprising administering to a patient in need thereof the polypeptide of any one of claims 1-4, the protein of any one of claims 5-9, the antibody or antigen-binding fragment of claim 10, the isolated nucleic acid molecule of claim 11, the expression vector of claim 12, the host cell of claim 13, the product prepared by the method of claim 14, or the pharmaceutical combination of claim 15; preferably, the disease is a tumor or an inflammatory disease;
preferably, the tumor is selected from glioblastoma, prostate cancer, blood cancer, B cell tumor, multiple myeloma, B cell lymphoma, B cell non-Hodgkin lymphoma, Hodgkin lymphoma, chronic lymphocytic leukemia, acute myeloid leukemia, cutaneous T cell lymphoma, T cell lymphoma, solid tumor, urothelial/bladder cancer, melanoma, lung cancer, renal cell carcinoma, breast cancer, gastric cancer and esophageal cancer, prostate cancer, pancreatic cancer, colorectal cancer, ovarian cancer, non-small cell lung cancer and squamous cell head and neck cancer.

18. The polypeptide of any one of claims 1-4, the protein of any one of claims 5-9, the antibody or antigen-binding fragment of claim 10, the isolated nucleic acid molecule of claim 11, the expression vector of claim 12, the host cell of claim 13, or the product prepared by the method of claim 14, or the pharmaceutical composition of claim 15 for use in preventing and/or treating a disease in an individual; preferably, the disease is a tumor or an inflammatory disease;
preferably, the tumor is selected from glioblastoma, prostate cancer, blood cancer, B cell tumor, multiple myeloma, B cell lymphoma, B cell non-Hodgkin lymphoma, Hodgkin lymphoma, chronic lymphocytic leukemia, acute myeloid leukemia, cutaneous T cell lymphoma, T cell lymphoma, solid tumor, urothelial/bladder cancer, melanoma, lung cancer, renal cell carcinoma, breast cancer, gastric cancer and esophageal cancer, prostate cancer, pancreatic cancer, colorectal cancer, ovarian cancer, non-small cell lung cancer and squamous cell head and neck cancer.
